(19) Europäisches Patentamt / European Patent Office / Office européen des brevets

(11) **EP 3 445 257 B1**

(12) **EUROPÄISCHE PATENTSCHRIFT**

(45) Veröffentlichungstag und Bekanntmachung des Hinweises auf die Patenterteilung:
**04.09.2019** **Patentblatt 2019/36**

(21) Anmeldenummer: **17720429.4**

(22) Anmeldetag: **24.04.2017**

(51) Int Cl.:
***A61B 17/16*** *(2006.01)*

(86) Internationale Anmeldenummer:
**PCT/EP2017/059670**

(87) Internationale Veröffentlichungsnummer:
**WO 2017/182673 (26.10.2017 Gazette 2017/43)**

(54) **CHIRURGISCHER FRÄSER**

SURGICAL MILLING CUTTER

FRAISE CHIRURGICALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2016 DE 102016107549**
**11.08.2016 DE 202016104435 U**

(43) Veröffentlichungstag der Anmeldung:
**27.02.2019 Patentblatt 2019/09**

(73) Patentinhaber: **Skajster Familienstiftung**
**9495 Triesen (LI)**

(72) Erfinder: **SKAJSTER, Tomasz Jan**
**44867 Bochum (DE)**

(74) Vertreter: **Lippert Stachow Patentanwälte**
**Rechtsanwälte**
**Partnerschaft mbB**
**Frankenforster Strasse 135-137**
**51427 Bergisch Gladbach (DE)**

(56) Entgegenhaltungen:
**WO-A1-93/09731       DE-A1-102004 040 581**
**DE-C1- 10 022 047    DE-C1- 19 634 484**
**US-A- 2 753 618      US-A1- 2004 133 209**
**US-A1- 2008 306 483**

**Beschreibung**

Die Erfindung betrifft einen chirurgischen Fräser, ausgebildet zum Abtragen von Knochen und/oder Knorpelgewebe, umfassend einen um eine Rotationsachse drehbaren und eine Längsachse definierenden Schaft, der einen Nenndurchmesser aufweist und ein drehfest mit einer Antriebseinheit verbindbares Proximalende sowie ein diesem Proximalende gegenübergelegenes Distalende aufweist, wobei an dem Distalende ein Fräskopf mit einer den Schaft umfänglich umschließenden und sich entlang der Längsachse des Schafts erstreckenden Fräsfläche ausgebildet ist, wobei die Fräsfläche begrenzt ist durch ein proximal gelegenes Fräsflächenproximalende sowie distal gelegenes Fräsflächendistalende und einen Fräsflächenmaximalradius aufweist, wobei die Fräsfläche auch proximal wirkend ausgebildet ist, wobei die Fräsfläche in distaler Richtung radial über den Nenndurchmesser des Schafts erweitert ausgebildet ist, wobei an dem Distalende eine Schutzanordnung ausgebildet ist, wobei die Schutzanordnung distal von dem Fräsflächendistalende ausgebildet ist und wobei die Schutzanordnung eine Distalauflagefläche aufweist. Die Begriffe proximal und distal sind in Bezug auf den Benutzer/Chirurg des Fräswerkzeugs als Referenzpunkt zu verstehen. Proximal bedeutet also in Richtung des Chirurgs und distal bedeutet von dem Chirurg abgewandt.

Mit derartigen Vorrichtungen können chirurgische Operationstechniken in offener, mikrochirurgischer, minimalinvasiver, endoskopischer oder in einer anderen Technik durchgeführt werden, welche im Rahmen der Verschaffung eines Zuganges zur Abtragung bzw. zum Abfräsen von Knochen eingesetzt werden oder im Wesentlichem aus diesen bestehen, wie z.B. bei knöcherner Dekompression. Solche Operationstechniken werden insbesondere in der Wirbelsäulenchirurgie und Neurochirurgie, aber auch in der Mund-Kiefer-Gesichtschirurgie, Hals-Nasen-Ohrenheilkunde und Orthopädie durchgeführt. Die Vorteile der Erfindung können jedoch auch die Verletzungsrisiken in den weiteren Anwendungsgebieten in der Medizin bzw. Chirurgie entschärfen.

Bei vielen chirurgischen Eingriffen müssen im Rahmen der Beschaffung eines Zuganges bestimmte Knochenfragmente entfernt werden, um den Einblick in ein Operationsfeld zu erweitern und weitere Schritte der Operation zu ermöglichen. Beim bekannten Operationsverfahren wird im Rahmen der Beschaffung des Zuganges zum Wirbelkanal das interlaminäre Fenster (enger spaltartiger Raum zwischen zwei Wirbelbogen=Lamina) nach Abschieben von autochtoner Rückenmuskulatur dargestellt und schrittweise mit unterschiedlich großen Kerrisonschen-Stanzen oder Fräsern erweitert. Beim Fräsen wird der zunächst gewählte Fräskopf mit Schneiden gegen eine Diamantfräse gewechselt, um das Verletzungsrisiko insb. ein Einfallen in die Tiefe zu minimieren. Man arbeitet langsam, vorsichtig Schicht für Schicht von oben nach unten möglichst in einer Ebene, wobei das Handstück mit der den Fräskopf führenden Hand stets unter Verwendung von Abdeckungstüchern am Patientenrücken abgestützt werden muss. Man arbeitet also von oben nach unten bzw. von außen nach innen.

Ein besonderer Fall hier ist eine knöcherne Dekompression des Spinalkanals, d.h. Entlastung der Nervenwurzel und ggf. auch des Rückenmarks durch Entfernung von einengenden knöchernen Anbauten. Dieser Eingriff kann entweder vorgenommen werden als selbstständige Operation oder bei vorliegender Spinalkanalstenose als Teil eines größeren Eingriffs beim Erweitern eines Zuganges i.S. der zuvor beschriebenen erweiterten interlaminären Fensterung oder Hemilaminektomie, z.B. Bandscheibenoperationen oder Tumoren sowie Fusions- oder Skolioseoperationen.

Üblicherweise werden zum Knochenabtragen entweder Knochenstanzen oder unterschiedliche, mit einem separaten Antrieb verbundene rotierende Instrumente als Fräser eingesetzt. Die Fräsflächen dieser Fräser können unterschiedliche geometrische Formen aufweisen. Im Wesentlichen bestehen diese alle aus einem drehfest mit einem Antriebswerkzeug verbindbaren Schaft, der ein proximales Ende ausgebildet zur drehfesten Verbindung mit eine Antriebswerkzeug aufweist, so dass das über den Schaft ausgeübte Drehmoment direkt auf den Fräskopf übertragen wird.

Bestehende chirurgische Fräser sind derart ausgebildet, dass die scharfe bzw. schneidende Fräsfläche den gesamten Fräskopf umfasst. Hauptsächlich werden kugelförmige Fräsköpfe verwendet, seltener werden Walzen-, Kegel-, Birnen-, Oliven- oder Flammenfräser gewählt.

Bei bekannten Fräsern werden Schneiden in Form von unterschiedlich konzipierten Verzahnungen verwendet oder eine Reibfläche aus aufgebrachten Kristallen, z.B. Diamanten. Bei der Rotation des Fräskopfes und einem gleichzeitigen direkten Kontakt zum Knochen unter Druck tangential zur Knochenoberfläche wird darunterliegender Knochen abgerieben bzw. abgefräst. Eine ständige Spülung ist notwendig, und zwar sowohl um den heiß werdenden Fräskopf zu kühlen als auch um das entstehende Knochenmehl aus der Fräskopffläche abzuspülen.

Recht häufig sind bei solchen Eingriffen die abzutragenden Knochenelemente direkt mit äußerst wichtigen anatomischen Strukturen benachbart. Dazu gehören unter anderem: Nerven, Wurzeln, Gefäße, Hirn-bzw. Rückenmarkshäute als auch darunterliegende Hirn- oder Rückenmarksgewebe. Eine Verletzung der o.g. Strukturen durch die Fräsfläche kann zu gravierenden und nicht immer reversiblen Schädigungen führen. Als schwerwiegende Folgen einer solchen Verletzung sind diverse Komplikationen u.a. Schlaganfälle, Nach-, Blutungen, Hirnnervenausfälle, Lähmungen, Seh-, Hör-, Miktions-, Defäkations-, Potenz- oder Sensibilitätsstörungen sowie andere neurologische Defizite und Behinderungen zu erwähnen; in seltenen Fällen können solche Komplikationen direkt oder indirekt zum Tode führen. Eine Verletzung von Hirn- bzw. Rückenmarkshäuten "Dura" (Dura mater spinalis et cranialis, nachfolgend "Dura" genannt) und hierdurch bedingter folgender Liquorausfluss (Nerven-, Hirnwasser, Gehirn-Rückenmarks-, Zerebrospinalflüssigkeit, Liquor cerebrospinalis) benötigt üblicherweise eine direkte Versorgung, welche die Operationsgesamtzeit verlängert und zusätzliche erhebliche Behandlungskosten verursacht. Dies kann auch zu einer verzögerten Wundheilungsstörung, bzw. Liquorkissen oder Liquorfisteln und infolgedessen zu chronifizierten Wundheilungsstörungen führen. Dadurch können sich die Infektionen in die Tiefe ausbreiten und z.B. Abszesse (Eiteransammlungen unter der Haut), Meningitiden (Hirnwasserentzündungen) entstehen, welche durch erneute Deckungsoperationen, Liquorableitungen und mit teuren Antibiotika über einen langen Zeitraum behandelt werden müssen.

Eine Duraverletzung ist die häufigste Komplikation bei Wirbelsäulenoperationen. Die nachfolgende Literaturauswahl erläutert das Ausmaß der Problematik und ist nicht abschließend zu Informationszwecken gedacht.

Laut Literatur tritt die Duraverletzung in 1-17% aller Wirbelsäulen-OPs auf, Guerin P at al. Injury, Int. J. Care Injured 43 (2012) 397-401. In mehreren Studien wird die Häufigkeit bei Operationen am LWS in Höhe von ca. 9% erwähnt, bei Revisionseingriffen in 3-27% der Fälle.

Bei einer Präsentation bei DGNC 2014 wurde eine inzidentelle Duraverletzung als Ursache einer moderaten Behinderung in 19,8% der Fälle, einer schweren in 19% und in 12% mit Gangunfähigkeit und Rollstuhlpflicht beziffert. In 30% aller Fälle ist eine Behinderung in 3 Jahren persistent, Boshara M at al. DGNC2014, doi: 10.3205/14dgnc541.

In den Vereinigten Staaten ist die Duraverletzung die zweithäufigste Ursache für die Klagen gegen Ärzte aufgrund von Komplikationen nach Wirbelsäulenoperationen, wobei die häufigste - Cauda-equina-Syndrom - ebenfalls unter anderem als Folge einer intraoperativen Verletzung beim Fräsen auftreten kann, Goodkin at al. Surg Neurol 1995; 43:4-14.

"Zwischen 2005 und 2013 nahm die Zahl der Wirbelsäuleneingriffe in Deutschland von knapp 327.000 auf rund 750.000 um mehr als das Doppelte zu". (wdr1)

In den USA wurden im Jahre 2011 alleine 460.000 Versteifungsoperationen laut AHRQ durchgeführt, die laut Marktwachstumsprognosen bis 2020 p.a. um mehr als >5% steigen werden.

Auch in den USA wurden die Mehrkosten für die Behandlung nach einer Duraverletzung in den Jahren 2008-2011 zusammengerechnet. Dabei wurden durchschnittlich Mehrkosten i.H.v. $7.638 pro Fall bei Eingriffen im Bereich der Halswirbelsäule und $2412 pro Fall bei Eingriffen im Bereich der Lendenwirbelsäule ermittelt.

Diese Daten zeigen, dass es sich um ein ernsthaftes medizinisches Problem handelt mit gravierenden sozialen und wirtschaftlichen Folgen.

Eine Verletzung einer der o.g. anatomischen Strukturen kann durch einen minimalen und äußerst kurzen Kontakt des schnell rotierenden scharfkantigen Fräskopfes mit einer der o.g. Strukturen entstehen. Eine ähnliche Schädigung kann auch durch die an der Spitze der Fräse durch die Scherkräfte erzeugte Wärme resultieren. Zusammenfassend: jede einzige ungeschickte Bewegung als auch Ausrutschen des schnell rotierenden Fräskopfes in die Tiefe (vertikal) oder zur Seite (horizontal) kann dramatische Folgen haben. Deswegen muss jeder Operateur stets sehr vorsichtig, unter ständiger Spülung und nur mit leichter Andruckkraft arbeiten, um solche Ereignisse zu vermeiden.

Die herkömmliche Geometrie der Fräsköpfe mit umfassenden scharfkantigen Arbeitsflächen schützt nicht vor solchen ungewünschten Verletzungen. Durch die Vorsichtsmaßnahme einer reduzierten Anpress- oder Andruckkraft wird die erzielbare Abtragsleistung signifikant gemindert, so dass die OP länger dauern.

Die gebotene Vorsicht und die eventuell notwendigen Interventionen z.B. wasserdichte Duranähte verlängern heutzutage gewissermaßen die Operationszeiten. Das hat medizinische (lineare Erhöhung der Infektionsrate in der Zeitfunktion der OP) als auch wirtschaftliche Konsequenzen.

In Literatur und in den Patentschriften werden sowohl die oben geschilderten Probleme erkannt als auch nach einer Lösung der o.g. Nachteile des Standes der Technik geforscht.

Die WO 2015/009810 schlägt eine spezielle Geometrie der Verzahnung der Fräsfläche vor. Diese Lösung kann jedoch bei einem kurzen, gelegentlichen frontalen Kontakt mit großflächigen Weichteilen, wie z.B. der harten Hirnhaut nur die Häufigkeit der o.g. Risiken reduzieren, kann aber weiterhin die zuvor beschriebenen Verletzungen nicht verhindern. Es fehlen auch die entsprechenden komparativen klinischen Studien bezüglich der tatsächlichen Reduktionsrate von Duraverletzungen /Liquorlecks. Die Folgen eines, insbesondere seitlichen Kontaktes zwischen dem rotierenden Fräskopf und Nervenwurzeln oder Gefäßen, welche deutlich empfindlicher vulnerabler als die harte Hirnhaut (Dura) sind, werden nicht diskutiert.

Die DE 10 022 047 C1 offenbart ein Instrument zum Schneiden einer Schädelkalotte, das am distalen Ende einen Ablösekörper aufweist. Diese Lösung ist jedoch nicht für alle Anwendungsgebiete einsetzbar, z.B. nicht geeignet für die Wirbelsäule. Die scharfe Arbeitsfläche endet hier vor dem Ablösekörper. Der sich zur Spitze hin konisch verjüngende Fräser eignet sich geometrisch vielleicht für punktuelle und radial arbeitende, schmale Eingriffe bzw. Schnitte - aber nicht für den aufwändigen Einsatz an der Wirbelsäule, weil bei derartigen Operationen viel mehr Knochengewebe entfernt werden muss. Insbesondere das Grundproblem des Abrutschens wird mit dieser Vorrichtung nicht gelöst, was insbesondere bei einem Nervenwasser befüllten Duralschlauch problematisch ist, das dieser unter ständigen Druck von 0-15 mmHg (0-2kPa) steht. Durch die Kompression mit der Schutzanordnung kommt sowohl zu einer minimalen Drucksteigerung als auch zu reaktiven Verwölbung des Duralschlauches - auch proximal zu den proximalen Auflagefläche der Schutzanordnung in der Richtung der Drehachse und des rotierenden Arbeitsteils.

Die bisher eingesetzten rotierenden Instrumente - Bohrer, Fräser und Fräsköpfe vermeiden die zuvor geschilderten Nachteile nicht zufriedenstellend.

Es gibt zwar einige spezielle Fräsköpfe mit einer stumpfen oder glatten Stirnfläche bzw. Kuppe, z.B. Nagelfräser für Diabetiker diverser Hersteller, einige zahnärztliche und endodontische Bohrer, einen Radiusfräser der Firma Metabowerke GmbH oder einen Osteophytenfräser der Firma Aesculap AG. Die eingesetzten Geometrien vermindern die Gefahr von Verletzungen nur unzureichend, wenn überhaupt.

Insbesondere schützen diese Vorrichtungen nicht vor einer Verletzung bei einer schrägen, nur leicht gekippten, d.h. nicht streng tangentialen oder sogar seitlichen Begegnung mit den weichteiligen anatomischen Strukturen. Diese Vorrichtungen können dem Operateur sogar ein falsches Sicherheitsgefühl vermitteln und so riskantes Verhalten ermutigen. Ein gattungsgemäßer Fräser ist aus der US 2004/133209 A1 bekannt. Weiterer Stand der Technik ist aus der DE 10 2004 040 581 A1 bekannt.

## Technisches Problem (Aufgabe)

Der Erfindung liegt das technische Problem (Aufgabe) zugrunde, die zuvor geschilderten Nachteile zumindest teilweise zu vermeiden und insbesondere einen chirurgischen Fräser vorzusehen und somit die vielen ungewollten Verletzungen bei medizinischen Eingriffen zu mindern oder gar zu vermeiden.

## Erfindung

Die Erfindung schlägt zur Lösung dieser Probleme die atraumatische Ausbildung des motorisch antreibbaren Fräsers vor, was bedeutet, dass der Fräser gewebeschonend ausgebildet ist und keine Verletzung verursacht. Bei der abstraktesten Ausführungsform wird dieses dadurch erzielt, dass der Fräskopf atraumatisch ausgebildet ist, dass die Fräsfläche auch proximal wirkend ausgebildet ist, dass die Fräsfläche in distaler Richtung radial über den Nenndurchmesser des Schafts erweitert ausgebildet ist, dass an dem Distalende eine Schutzanordnung ausgebildet ist, dass die Schutzanordnung distal von dem Fräsflächendistalende ausgebildet ist, dass die Schutzanordnung eine Distalauflagefläche und einen Schutzanordnungsmaximalradius zur Bildung eines Schutzrings aufweist, der den durch den Fräsflächenmaximalradius definierten Fräsflächenmaximalumfang kreisringförmig umschließt, so dass die Schutzanordnung bei der Rotation des Fräsers eine sich von dem Schutzanordnungsmaximalradius bis zum Fräsflächendistalende erstreckende, umfänglich umgebende Schutzzone um die Fräsfläche definiert.

Durch diese distal von der Fräsfläche an dem Fräser angeordnete Schutzanordnung werden die unter (distal) und seitlich (radial) von der Fräsfläche gelegenen Gewebepartien, z.B. Dura, Nervenwurzel, Gefäße von der scharfkantigen und nur proximal wirkenden Fräsfläche abgeschirmt. Aufgrund der geeigneten Festlegung der variablen Maße aller Teile des Fräskopfes wird die Schutzanordnung mit den Sicherheitsabständen zu den benachbarten Weichteilen und die anatomisch bedingten Einsatzmöglichkeiten sowie die Handhabung festgelegt.

Die Fräsfläche ist erfindungsgemäß auch proximal wirkend ausgebildet, womit gemeint ist, dass diese ausgebildet ist, in der proximalen Richtung zu fräsen bzw. zu schneiden, was nicht ausschließt, dass diese auch für radiale Schnitte eingesetzt wird. Durch diese Ausgestaltung kann erstmalig Zug auf den Schaft ausgeübt werden.

Die Schutzzone ist ein bei der Rotation des Fräsers im Betrieb erzeugter virtueller Raum im Sinne eines Rotationskörpers, welcher proximal begrenzt wird durch das proximale Ende der Fräsfläche (Fräsflächenproximalende) und distal durch den Schutzring und dessen Schutzanordnungsmaximalradius R2 begrenzt wird, welcher den Fräsflächenmaximalradius R1, also die maximale radiale Ausdehnung der Fräsfläche von der Rotationsachse, kreisringförmig umschließt.

Die Entwicklung der Erfindung beruht auf der Erkenntnis, dass durch eine korrekte und bedarfsgerechte geometrische Gestaltung des Schutzrings kein Verletzungsrisiko durch Eindringen von Gewebe proximal des Schutzrings besteht bei Routinearbeiten und ein Kippen des Fräsers ermöglicht wird. Versuche haben ergeben, dass der Schutzanordnungsmaximalradius R2 mindestens die Größe des Fräsflächenmaximalradius R1 zzgl. der Toleranz zzgl. das Zehnfache des Rundlaufs aufweisen sollte, aber auch deutlich größer gestaltet sein kann. Als unsicher hat sich hingegen eine Zone um den Fräser herum erwiesen, welche sich innenseitig von der jeweiligen, in Längserstreckungsrichtung ausgebildeten Radius des Fräser zzgl. der oberen Grenzabweichung zzgl. des Rundlaufs beträgt.

Die konkrete geometrische Ausgestaltung der Schutzanordnung kann bedarfsorientiert angepasst werden, also die Größe, Länge und Breite bzw. Umfang und Grad der Konvexität der Schutzanordnung und der somit realisierte distale bzw. radiale Sicherheitsabstand zu den angrenzenden Weichteilen, die sich vor Distalauflagefläche und um die Distalauflagefläche der Schutzanordnung befinden.

Der Schutzring umschließt den Fräsflächenmaximalumfang kreisringförmig umfänglich und bildet somit den Bereich der maximalen radialen Ausdehnung bzw. den Bereich mit dem maximalen Umfang und hält somit das umgebende Gewebe von der Fräsfläche fern.

Die minimale Größe von dem Schutzanordnungsmaximalradius R2min beträgt:

$$R2min = \frac{L \times K}{\tan \beta 1} - \frac{r - r \times \tan\beta 1}{\tan\beta 1} + R1 + T + 10 \times RO$$

Dabei sind:

R2min: minimaler Schutzanordnungsmaximalradius
L: Länge der Schutzanordnung entlang der Rotationsachse
K: Koeffizient der zweckbestimmten Geometrie der Schutzanordnung
$\beta_1$: Keilwinkel der proximalen Keilfläche bzw. Auflagefläche der Schutzanordnung
r: Radius der radial äußeren Abrundung der Schutzanordnung
R1: Fräsflächenmaximalradius des Fräsers
T: obere vorgegebene Grenzabweichung
RO: vorgegebener Rundlaufwert

$\beta_1$, L, r, R1 werden in der Längsquerschnittebene (entlang der Rotationsachse) des Fräsers gemessen. T, RO werden für das konkrete Fertigungsverfahren aus den allgemeinen Normen (DIN, EN, ISO, ANSI) oder Werknormen bestimmt. Der Koeffizient der zweckbestimmten Geometrie (K) wird durch die Zweckbestimmung des Produktes (Technische Dokumentation) und aus den wissenschaftlichen Daten (Anatomie) berechnet.

Somit besteht die Möglichkeit, für einen speziellen Anwendungsfall (für einen bestimmten Eingriff für einen bestimmten Patienten anhand seiner Bildgebung) die individuelle Werte auszurechnen und einen passenden Fräser auszuwählen. Der anhand dieser Formel errechnete Wert R2min liefert somit einen minimalen Schutzanordnungsmaximalradius, welcher das Risiko einer Duraverletzung unter 0,01% absenkt, womit diese also für alle praktischen Fälle ausgeschlossen ist.

Die glatte, also kantenfrei ausgebildete Schutzanordnung bildet am Distalende des Fräsers die Distalauflagefläche und mit dem Schutzanordnungsmaximalradius einen Schutzanordnungsmaximalumfang, der den Maximalumfang der Fräsfläche außenseitig als Schutzring, also kreisringförmig einfasst bzw. umschließt, also als geschlossener und übergangsloser Kreisring. Abhängig vom Anwendungsfall beträgt die Länge des Schutzrings in Längserstreckungsrichtung des Fräser vorzugsweise zwischen 0,25 und 4,5 mm, wobei sich eine Länge von 1 bis 4 mm bei Operationen an der Lendenwirbelsäule und eine Länge von 0,5 bis 2,5 mm bei Operationen der Halswirbelsäule als zweckmäßig herausgestellt haben. Eine Länge zwischen 0,25 bis 2,5 mm ist besonders für Eingriffe an der Schädelbasis, in der HNO-Chirurgie (Mittelohr-OPs) und bei den MKG-Eingriffen geeignet.

Die Länge L der Schutzanordnung ist durch die anatomischen Verhältnisse eingeschränkt. Zwischen Knochen und Dura erstreckt sich das Epiduralraum. Dieses ist einer hohler, spaltförmiger Raum unter den Knochen, in welchen die Schutzanordnung des Fräsers sicher eingeführt werden kann, ohne die Dura versehentlich zu komprimieren und zu beschädigen. Im Bereich der Lendenwirbelsäule beträgt dieser Spalt mittig sogar 2 bis 9 mm, seitlich wird der Spalt wesentlich schmaler - je nach Höhe beträgt dieser nur 0,8 bis 2,5 mm. Bei einer Spinalkanalstenose sogar noch weniger.

Die Entfernung zwischen der Nervenwurzel und dem Knochen beträgt 1,9 bis 4,1mm oberhalb der Wurzel. Die Wurzel kann mit der Schutzanordnung des Fräsers im Wurzelkanal noch um 1,5 bis 3,1 mm tiefer sanft abgeschoben werden.

Die Länge L der Schutzanordnung soll anhand dieser Werte angepasst oder aus der präoperativen Patientenbildgebung errechnet werden. Die zweckmäßigen Maximalwerte L liegen in den nachfolgend angegebenen Bereichen:

| Halswirbelsäule | 2,5mm |
|---|---|
| Brustwirbelsäule | 3mm |
| Lendenwirbelsäule | 4,5mm |

Die Distalauflagefläche der Schutzanordnung bildet somit - zumindest teilweise - die Auflagefläche des Fräsers in der Vertikalen und der Schutzanordnungsmaximalumfang bestimmt den seitlichen, also horizontalen Schutzraum um die Fräsfläche herum. Durch die Schutzanordnung wird beim Fräsen zwischen der scharfen Fräsfläche und den seitlich daneben (horizontal) und unterhalb, also distal bzw. (vertikal) von der Fräsfläche angrenzenden anatomischen Strukturen eine virtuelle dreidimensionale Sicherheitszone gebildet, die das den Operationsbereich umgebende Gewebe von der Schneidfläche weg hält und somit ungewollte Verletzungen von diesem Gewebe wirksam verhindert, gleichzeitig aber eine uneingeschränkte Abtragsleistung bei guter Handhabung ermöglicht. Die Schutzanordnung bietet somit erstmalig horizontalen und auch vertikalen Schutz des umgebenden Gewebes.

Um die Fräsfläche herum befindet sich am vorderen Distalende die Schutzanordnung, welche einen Kontakt mit dem sensiblen Gewebe verhindert - egal wo sich dieses befindet - sowohl vertikal also unter dem Schutzgürtel als auch horizontal also daneben (im Umkreis).

Da man mit dem vorgeschlagenen Fräser anders als bisher arbeiten kann, wird somit auch beim Ausrutschen eine unbeabsichtigte Verletzung in die Tiefe wirksam vermieden. Im Gegensatz zu bekannten Operationsverfahren kann der Fräser beim Einsatz des erfindungsgemäßen Fräsers peu à peu tangierend zur Knochenkante herausgezogen und nicht reingedrückt werden. Damit verläuft die resultierende Kraft stets tangential zum Knochen, also seitlich und eher rückwärts, also proximal nach außen von dem OP-Feld, d.h. in der Richtung des Handstücks.

Da beim Herausziehen so gut wie keine zu verletzenden Strukturen vorliegen, kann der Fräser mit deutlich mehr Kraft eingesetzt werden als bei bekannten Operationsverfahren. Dieses reduziert die OP-Dauer erheblich. Da die Schutzanordnung die Selbstzentrierung des Fräsers bewirkt, ist die Gefahr eines ungewollten Ausrutschens ausgeschlossen. Insofern besteht keine Einschränkung bei der Kraftanwendung und man kann die submaximale zulässige Anpress-Kraft tangential zur Knochenkante (ungefähr Senkrecht (+/- 30 Grad) zu der Längsachse) verwenden. Submaximal ist i.S. von Fingerspitzengefühl zu verstehen, damit ein Knochenstück nicht durch die Kraftanwendung selbst frakturiert bzw. ausgebrochen wird. Die gefühlte subjektive Knochendichte variiert zwischen abhängig vom Patienten von "steinhart" bis "knetgummi-artig". Bei ausgeprägter Osteoporose bedarf dieses einer sehr zarten Handhabung.

Der erfindungsgemäße Fräser erlaubt das Arbeiten mit fast der dreifachen Kraftaufbringung, womit etwa eine gleiche Verbesserung der Schnittleistung, Schnittkraft und Schnittgeschwindigkeit einher geht. Mit Hilfe eines Dynamometers wurde die Höhe der aufgebrachten Kraft durch den Chirurgen von bekannten Fräsern mit dem erfindungsgemäßen Fräser verglichen. Bislang konnte ein Chirurg oberflächlich mit einer Kraft von max. 30N arbeiten, musste diese aufgebrachte Kraft dann aber aus Sicherheitsgründen bis auf 5 Newton reduzieren. Mit dem vorgeschlagenen Fräser kann der Chirurg hingegen kontinuierlich mit einer Kraft von bis zu 80N arbeiten, und zwar unabhängig von der Tiefe. Die Schutzanordnung ist vorzugsweise ausgebildet, um die Distalauflagefläche und/oder den Schutzanordnungsmaximalumfang des Fräskopfes zu vergrößern, bei bestimmten Aufführungsformen um das 1,5- bis 3-fache. Bei erhaltener Abtragsleistung werden durch diese Ausbildung alle distal von und neben dem Fräskopf befindlichen Weichteile geschont bzw. geschützt, und zwar ohne Einschränkung von Bewegungsfreiheit für den Operateur beim Fräsen bzw. bei der Operation.

Die Schutzanordnung schützt beim Fräsen in allen üblichen Arbeitswinkeln gegen die zuvor beschriebenen unbeabsichtigten Verletzungen des den Operationsraum umgebenden Gewebes, welche grundsätzlich abhängig sind von der Eingriffsart, dem Zugangsweg und den individuellen anatomischen Bedingungen, z.B. der Dicke der Fettschicht, dem variablen Verlauf der Gefäße und Nerven usw. So kann z.B. an der Wirbelsäule bei dorsalem Zugang von max. -45 Grad medial bis max. 90 Grad lateral und max. -90 Grad bis max. 90 Grad in der kranio-kaudalen Ebene gearbeitet werden.

Eine vorzugsweise glatte bzw. glattpolierte Ausbildung der Schutzanordnung an dem Distalende des Fräsers schützt bei einem gelegentlichen oder längeren Kontakt beim Fräsen die besonders gefährdeten, sensiblen Strukturen vor einer Verletzung und ermöglicht zudem eine sichere und wesentlich schnellere Durchführung des Eingriffes.

Aufgrund der glatten Schutzanordnung kann der Fräser zudem schonend und ohne Verletzungsgefahr in das Gewebe oder zwischen Knochen eingeführt und das umgebende Gewebe mit diesem beiseite geschoben werden. Dies wird insbesondere in einem modernen minimal-invasiven Verfahren begrüßt, weil durch die o.g. Konstruktionsmerkmale sowohl die Hauteröffnung als auch die zugangsbedingte Gewebeschädigung mit geringerem Druck ausgeübt werden muss. Dieses beschleunigt die Wundheilung und reduziert die Häufigkeit von Wundheilungsstörungen und anderen infektiösen Komplikationen. Die minimalinvasive Operationstechnik erlaubt es dem Patienten, früh postoperativ zu mobilisieren (infolgedessen verringert sich das Risiko von Thrombosen/Embolien, Lungenentzündung), postoperative Schmerzen sind weniger stark ausgeprägt, so dass auch weniger Schmerzmittel eingesetzt werden müssen.

Der maximale Durchmesser der Schutzanordnung und damit dessen Maximalumfang bestimmen den seitlichen Sicherheitsabstand sowie die mögliche Verwölbung des Gewebes von der Seite. Der eingangs erwähnte, mit Nervenwasser befüllte Duralschlauch wird durch die Schutzanordnung zwar ebenfalls komprimiert, jedoch werden Beschädigungen aufgrund der durch die Schutzanordnung verwirklichten reduzierten Flächenpressung wesentlich unwahrscheinlicher, so dass der Duralschlauch sich sogar proximal von dem Schutzring zu der Schutzanordnung ohne Beschädigung verwölben kann.

Bei ebenen Operationen, also nur in der Horizontalen bzw. seitwärts gerichtet, also ohne Änderung der Eindringtiefe, wirkt die Schutzanordnung zudem insofern zentrierend bzw. positionierend, weil der Fräser durch diese an den umgebenden Knochen geführt wird. Der abgerundete Rand der Schutzanordnung schützt dabei stets vor Verletzungen bei einem seitlichen Kontakt.

Die folgenden Vorteile werden durch die Erfindung erzielt:

• Es wird ausschließlich und gezielt die bestimmte Knochenfläche abgefräst.

• Alle vor und neben dem Fräskopf gelegenen Gewebestrukturen werden durch die Schutzanordnung geschont.

- Dieses ermöglicht bei suffizienter Spülung zwecks Kühlung und Schmierung einen gelegentlichen oder dauerhaften Kontakt mit dem vulnerablen umgebenden Gewebe beim Fräsen.

- Durch die glatte Schutzanordnung werden die anatomischen Strukturen schonend beiseitegeschoben und/oder ferngehalten.

- Der Fräser wird bereits durch die kantenfreie Schutzanordnung, insbesondere durch den Schutzring stets in der Sollposition gehalten, ist also selbstzentrierend ausgebildet, wodurch weder das Ausrutschen noch ein vertikales Eindringen in die Tiefe auftreten kann. Die Abstandshaltung zum umgebenden Gewebe wird durch den Schutzring verbessert.

- Somit kann ohne Risikoanstieg einer intraoperativen Verletzung bei der Anwendung eines bis zu dreimal höhere Anpresskraft bzw. Vorschubkraft eingesetzt werden, wobei die Temperatur bei Erhöhung der aufgebrachten Kraft langsamer ansteigt; suffiziente kühlende und schmierende Spülung, vorzugsweise mit einer Kochsalzlösung vorausgesetzt.

- Dieses ermöglicht eine bis zu 3-fach höhere Abtragsleistung beim Fräsen, was die Dauer eines Eingriffs verkürzt. Obwohl dieses zwar stark vom Operateur und vom Einzelfall abhängt, wurden in den meisten Fällen Zeitersparnisse von 25 bis 50 Prozent und mehr erzielt, so dass die durchschnittliche OP-Dauer von ca. 45 bis 60 Minuten auf ca. 15-25 Min reduziert wurde.

Aufgrund der sich an die Fräsfläche distal einfassenden glatten Schutzanordnung mit abgerundeten Kanten kann der Fräser direkt parallel zum abzufräsenden Knochenrand entlang der Einsatzstelle am Knochenrand geführt werden, so dass deutlich größere als bisher übliche Durchmesser an Fräsköpfen eingesetzt werden können. Dieses ermöglicht eine die bis zu 3-fach höhere Abtragsleistung und reduziert somit die OP-Dauer. Wegen der höheren Wärmekapazität des größeren Fräsers mit der konsekutiv größeren Schutzanordnung steigt die Temperatur beim Fräsen bei deutlich langsamer als bei herkömmlichen Fräsern.

Der Schutzring bildet somit auch eine proximale Auflagefläche, die vorzugsweise tellerförmig ausgebildet ist und ein sanftes Führen des Fräsers entlang einer zu bearbeitenden Knochenkante ermöglicht. Die Proximalseite der Schutzanordnung bzw. des Schutzrings kann also zur Führung mit der Knochenkante, bzw. Knochenoberfläche in Kontakt treten und stützt sich dabei am Knochenrand ab.

Zur Vergrößerung dieser Anlagefläche kann die Schutzanordnung einen atraumatisch ausgebildeten Zwischenabschnitt zwischen dem Fräsflächenmaximalradius und Schutzanordnungsmaximalradius umfassen, welcher bedarfsgerecht beliebig ausgebildet sind kann, z.B. als gerade, konvex oder konkav gekrümmt oder auch als Freiformfläche.

Ausführungsform sehen einen Relativersatz zwischen der Rotationsachse des Fräsers (Fräserrotationsachse) und einer Symmetrieachse der Schutzanordnung vor, die dann aber nicht drehfest mit dem Fräser verbunden ist, wodurch eine verbesserte Anpassungsfähigkeit insbesondere in engen anatomischen Verhältnissen realisiert wird.

Um eine besonders große Schutzanordnung mit besonders großem Hebelarm und eine gute Schutzwirkung bereitzustellen hat sich die pilzförmige Ausgestaltung der Distalauflagefläche als besonders zweckmäßig erwiesen. Pilzförmig bedeutet, dass beide konvexe oder gerade Flächen der Schutzanordnung einen ähnliche Form wie ein Hut eines Pilzes aufweist und einen umfänglichen Keil mit einer abgerundeten Spitze bildet - entweder durch die virtuellen Verlängerungslinien der Flächen selbst oder über die Tangenten an beiden Flächen, die bevorzugt konvex gekrümmt sind.

Neben der Ausbildung der Schutzanordnung als Schutzring, welcher zunächst nur den Maximalumfang der Fräsfläche umfänglich atraumatisch einfasst, ist aber auch die Ausbildung als Schutzkappe möglich, welche neben dem Maximalumfang auch das Distalende atraumatisch umschließt. Diese Schutzkappe umschließt das Distalende bis zum Maximalumfang vorderseitig ein bis zum Maximalumfang der Fräsfläche, fasst also das vordere Distalende zumindest kappenartig bis zum Fräsflächenmaximalradialumfang ein bzw. umschließt dieses.

Die Schutzkappe weist vorzugsweise einen positiven Konvexitätsgrad auf, ist also sphärisch ausgebildet, sie kann aber auch zentral flach oder konkav ausgebildet sein. Die distale Fläche der Schutzkappe bildet somit die Distalauflagefläche des Fräsers. Die Distalauflagefläche der Schutzanordnung wird somit leicht an dem Gewebe abgefedert, bildet also neben der Distalauflagefläche einen leicht elastischen zweiten Stützpunkt mit dem in dem Gewebe, welcher den Fräser zusätzlich stabilisiert und schwingungsdämpfend wirkt.

Bei der bevorzugten konvexen Ausbildung bildet die Schutzkappe somit die Distalauflagefläche mit der Gestalt eines Kugelsegments. Dieses bietet für das umgebende Gewebe vorwiegend vertikalen, also frontseitigen Schutz, aber auch horizontalen Schutz bei schräger Ausrichtung des Fräskopfs. Auf diese Weise werden alle distal der Schutzanordnung gelegenen taktilen Strukturen geschont, auch wenn ein direkter frontaler Kontakt länger auftritt; bei ausreichender Spülung, Kühlung und Schmierung sogar auf Dauer.

Um von dem abzufräsenden Knochenteil die taktilen Weichteile schonend abzuschieben und eine gute Anbindung zur Knochenoberfläche zu ermöglichen, hat sich eine kugelsegmentförmige bzw. pilzförmige Gestaltung dieser Schutzkappe als besonders zweckmäßig herausgestellt.

Eine Verbesserung der Schutzwirkung kann durch vorsehen eines Schutzgürtels erzielt werden, der die Oberfläche des Fräsers auch oberhalb des Fräsflächenmaximalradius gürtelartig umgibt. Dieser Schutzgürtel kann unterschiedlich ausgebildet sein, so z.B. in Form eines schmalen, glatten oder beschichteten äußeren Umfangsgürtels, welcher die Weichteile wirksam vor einem seitlichen Kontakt mit der Fräsfläche schützt.

Die Fräsfläche geht vorzugsweise übergangslos, jedenfalls aber kantenlos in den Schutzgürtel über, der sich proximal von dem Fräsflächenmaximalradius erstreckt, vorzugsweise um 5 bis 10 Prozent.

Der Schutzgürtel weist dabei eine geringfügig größere Radialausdehnung als der Fräsflächenmaximalradius des Fräsers am Ende der Fräsfläche auf, so dass diese das untere Distalende der Fräsfläche gürtelartig umschließt. Der flach und randlos ausgebildete Schutzgürtel umschließt also den Maximalumfang der Fräsfläche und bildet so einen den Maximalumfang umfänglich umschließenden Schutzgürtel bzw. -ring.

Der Radius bzw. die Krümmung der Schutzkappe kann angepasst an den Anwendungsfall kleiner oder größer sein als der Radius des Fräsflächenmaximalumfangs. Vorzugsweise ist die Schutzkappe kugelsegmentförmig, kann aber auch eine komplexe und eine nicht kugelförmige Geometrie aufweisen. Eine kleinere Ausbildung erleichtert das Eindringen unter den Knochenrand, wohingegen eine größere Ausbildung den aufgespannten Sicherheitsraum durch Wegschieben des umgebenden Gewebes vergrößert. Bevorzugt ist eine Ausgestaltung, bei welcher der Radius der Schutzkappe deutlich größer ist als der Radius des Maximalumfangs, bevorzugt ist dieser etwa 5 bis 10 % größer als der Fräsflächenmaximalradius bzw. -umfang. Damit bildet die Schutzkappe bei der bevorzugten Ausführungsform eine distal vorstehende, pilzförmige Kugelsegmentfläche, die sich distal zumindest von dem Fräsflächenmaximalumfang bis zu einem distalen Vorderende erstreckt und Distalauflagefläche umfasst.

Die Schutzwirkung der Schutzanordnung kann zudem durch eine widerstandsreduzierte Ausgestaltung verbessert werden. Dabei kann die Schutzanordnung ganz aus einem solchen Material bestehen, z.B. PTFE/Teflon® oder auch nur teilweise mit diesem beschichtet sein, so dass die Schutzanordnung einen minimalen Widerstand darstellt. Vorzugsweise wird dieses realisiert durch Glätten oder Polieren. Auch eine keramische, metallische oder kunststoffhaltige Beschichtung (z.B. PTFE/Teflon®) auf der Schutzanordnung ist möglich, wobei aber darauf zu achten ist, dass die Fräsfläche nicht beschichtet ist.

Die Ausgestaltung und die Gestaltung der scharfkantigen und als Rotationskörper ausgebildeten Fräsfläche sowie die Arten der Verzahnung sind bedarfsgerecht wählbar. Diese kann z.B. halbkugel-, kegel- oder spindelförmig ausgebildet sein, wobei auch jegliche konvexen oder konkave Varianten als auch Sonderformen (z.B. sanduhrförmig) möglich sind. Bevorzugt umfasst die Fräsfläche Schneiden, die sich parallel oder schräg zur Längsachse des Fräskopfes auf der Fräsfläche erstrecken. Die Fräsfläche kann auch schraubenförmige oder geradlinige Rillen umfassen, und es können Spanwinkel vorgesehen sein, die ein reibungsloses Schneiden von Kortikalisknochen ermöglichen, wobei diese Winkel gewöhnlich positiv sind und im Allgemeinen etwa 5° bis 15°, besonders bevorzugt 7° betragen. Bei einem Freiwinkel von vorzugsweise 15° ergibt sich dadurch ein Keilwinkel der Schneidkante von etwa 60-70 °. Bei einer weiteren Ausführungsform sind mit unterschiedlichen Methoden kleine Kristalle (z.B. Korund, Zirkon, Diamant) auf der Fräsfläche aufgebracht.

Der Drallwinkel der Fräsfläche liegt vorzugsweise im Bereich zwischen 0°-35°. Für Eingriffe an den Wirbelknochen hat sich ein Linksdrallwinkel von vorzugsweise 15° zur Erzielung einer optimalen Abtragsleistung als besonders zweckmäßig erwiesen.

Der vorgeschlagene Fräser kann auch in den anderen technischen Gebieten eingesetzt werden, und zwar in allen Einsatzgebieten, in denen man einerseits mit einer scharfen Fräse arbeiten muss, gleichzeitig aber in der Umgebung des Fräskopfes befindliche Strukturen geschont werden müssen. Neben der Humanmedizin ist dieses ebenfalls in der Zahnmedizin, aber auch in der Tiermedizin einsetzbar. Möglich sind aber auch Einsatzgebiete in anderen technischen Gebieten, z.B. im Maschinenbau, bei Juwelierarbeiten, im Modellbau, in der Fußpflege u.ä.

Die distal angeordnete Oberfläche der Schutzanordnung ist glatt ausgebildet, was bevorzugt durch Polieren erfolgt. Ferner ist es zweckmäßig, wenn Kanten abgerundet sind und das untere Distalende der Fräsfläche proximal von dem lateralen Rand des Schutzgürtels endet.

Bei der bevorzugten Ausbildung besteht zumindest der Fräskopf des Fräsers aus Stahl, einer Stahllegierung, Hartmetall, Kunststoff, Keramik oder geeigneter Kombinationen dieser Materialien.

Der Fräser ist insbesondere als Drehteil ausgebildet, wobei bei der Herstellung aus einem Vollmaterial zunächst die distale Schutzanordnung gedreht wird, sodann in distaler Richtung ein Konus im Bereich der Schneidfläche eingestochen wird und schließlich auf diesem Konus die Schneidfläche ausgebildet wird.

Bevorzugt verbreitert sich der Radialabstand der Fräsfläche von dem Proximalende (Fräsflächenproximalende) bis zum Distalende der Fräsfläche (Fräsflächendistalende), wobei beliebige Ausgestaltungen der Fräsfläche angepasst an den jeweiligen Anwendungsfall möglich sein, insbesondere ein progressiv zum Distalende ansteigender oder aber auch ein komplexer Flächenverlauf. Diese proximal wirkende Ausgestaltung der Fräsfläche ermöglicht erstmalig das Fräsen/Schneiden in proximaler Richtung. Dieses wird insbesondere durch eine konisch sich distal in Längserstreckungsrichtung erweiternde Ausbildung der Fräsfläche realisiert, z.B. mittels eines kegeligen Fräskopfs, der sich vom Fräsflächenproximalende bis zu einem Fräsflächendistalende konisch erweitert und die Mantelfläche der Fräsfläche mit einer quer zur Längsachse verlaufenden Ebene einen Profilwinkel σ kleiner als 90 Grad einschließt. Die Konizität muss nicht an jeder Stelle dieselbe Steigung aufweisen, sondern die Erfindung ermöglicht beliebige Ausgestaltungen eines sich distal erweiternden, rotationssymmetrischen Fräskopfs. Erfindungsgemäß ist vielmehr angepasst an den Anwendungsfall auch eine konkave bzw. konvexe Ausbildung der sich in distaler Richtung radial erweiternden Fräsfläche realisierbar. Gerade diese erweiternde Ausbildung der Fräsfläche ermöglicht die durch die proximal gerichtete Arbeitsweise quasi "auf dem Rückweg" den Knochen abzufräsen.

Durch ein Kippen des Fräser kann ferner eine größere Fläche des Knochens bearbeitet werden.

Dieses ermöglicht erstmalig ein "Undercutting", was bedeutet, dass mehr von der distalen Knochenschicht als von der proximalen Knochenschicht abgetragen wird und zumindest eine dünne, knöcherne Brücke an der proximalen Knochenoberfläche bestehen bleiben kann.

Ferner kann der Fräser auf der Distalauflagefläche der Schutzanordnung aufsitzend gekippt und bewegt werden, wobei die Entfernung von der vormaligen Drehachse des Schafts zum jeweils sich im Kontakt mit dem Knochen befindlichen Punkt der Fräsfläche als Hebelarm kraftverstärkend wirkt. Auf diese Weise muss der Chirurg für zur Erzielung des gewünschten Vorschubs deutlich weniger eigene Kraft aufbringen, was insbesondere bei langen und komplizierten Operationen von Vorteil ist und einer Ermüdung der Chirurgs entgegenwirkt. Zusammenfassend muss der Chirurg also weniger Kraft aufbringen, kann aber gleichzeitig mit mehr Vorschub arbeiten.

Eine wechselnde Anpresskraft zwischen der Fräsfläche und dem Knochen hat sich als besonders zweckmäßig für das Fräsen erwiesen, weil dieses den Abtransport des abgetragenen Knochenmehls und dessen Ausspülen begünstigt. Ferner wird aufgrund des durch die Bewegung periodisch auftretenden Spalts die visuelle Kontrolle für den Chirurg erheblich verbessert. Auch eine durch wechselnde Belastung der Arbeitsfläche erzeugte Schwingung hilft die keilförmige Schutzanordnung schrittweise unter den Knochen zu schieben.

Neben einstückig und vorzugsweise aus Metall gefertigten Fräsköpfen liegt auch die zweiteilige Ausgestaltung im Rahmen der Erfindung. Dabei kann die gesamte Schutzanordnung umfassend Schutzring und Schutzkappe mit der Distalauflagefläche oder nur der Schutzring mit dem Fräser verbindbar ausgebildet sein, womit der Fräser dann die Distalauflagefläche aufweist. Bei einem mit dem Fräser verbindbarem Schutzring hat sich die Ausbildung als torusförmiger Schutzring als besonders zweckmäßig erwiesen.

Bei der zweiteiligen Ausführungsform ist der Fräskopf mit der Schneidfläche vorzugsweise aus Metall gefertigt, insbesondere als Drehteil, und die mit dem Fräskopf verbindbare Schutzanordnung kann aus einem anderen Materialbesthen.

Die somit austauschbare Schutzanordnung kann insofern separat und z.B. im Spritzgussverfahren hergestellt werden.

Es sind verschiedene technische Möglichkeiten der Verbindung zwischen Fräskopf und Schutzanordnung möglich. Bei der bevorzugten Ausführungsform ist an dem Fräskopf insbesondere distal von der Fräsfläche mit dessen Maximalumfang ein Sitz für die Schutzanordnung vorgesehen, z.B. ausgebildet als Rille oder Hinterschnitt.

Eine Ausführungsform zur Befestigung der Schutzanordnung an dem distalen Endes des Fräskopfs umfasst eine Schraubverbindung. Dabei sind grundsätzlich zwei unterschiedliche Ausgestaltungen möglich, nämlich eine "männliche" Version und eine "weibliche" Version. Die "männliche" Version umfasst am distalen Ende unterhalb der Fräsfläche einen Gewindestift, der entweder direkt mit der Schutzanordnung verbindbar ist oder mittels einer zusätzlichen Mutter, die in einer komplementär ausgebildeten Aufnahme der Schutzanordnung so aufgenommen ist, dass bei der OP keine Kanten mit dem umliegenden Gewebe in Berührung kommen können. Die "weibliche" Version umfasst hingegen in dem Distalende des Fräskopfs ein Innengewinde, in welches ein die Schutzanordnung haltendes Befestigungselement, z.B. ein Gewindebolzen mit Innensechskant, eindrehbar ist.

Die Gewindepaarung ist vorzugsweise so gestaltet, dass diese bei der Rotation des Fräsers um die Rotationsachse ein Anziehen der Gewindeverbindung bewirkt. Damit tragen die bei der Benutzung auftretenden Drehmomente aktiv zur Sicherheit bei und verhindern ein Lockern bzw. Losdrehen der Schutzanordnung vom Fräskopf.

Der Vorteil der getrennten Ausbildung der Schutzanordnung von dem Fräser liegt in der besseren Sterilisationsmöglichkeit und der Wiederverwendbarkeit. So kann die Schutzanordnung aus einem Kunststoff gefertigt sein, dass nur einmal oder nur mehrere Male verwendbar ist, wohingegen der Fräser mit dem Fräskopf vorzugsweise aus Edelstahl gefertigt ist, der sterilisiert bzw. resterilisiert werden kann. Es hat sich ferner gezeigt, dass komplexe geometrische Gestaltungen des Fräskopfes mit Fräsern und Schleifscheiben einfacher zu fertigen sind, wenn die Schutzanordnung nachträglich an dem Fräskopf befestigt wird.

Die Schutzanordnung, ob nun einteilig mit dem Fräser ausgebildet oder als mit dem Fräser verbindbarer Schutzaufsatz ausgebildet, kann an den Anwendungsfall angepasst ausgebildet sein, z.B. als sphärisches Kugelsegment, als Ring, als teilweise abgeflachte Kugel, als torusförmiger Ring, als Scheibe, als Scheibe mit einer konkav gewölbten Schutzfläche am distalen Vorderende.

Die Schutzanordnung kann somit Abschnitte mit mehreren Geometrien bzw. unterschiedliche Geometrieabschnitte zur Realisierung unterschiedlicher Funktionen umfassen. Ein erster Abschnitt umfasst den Schutzring, der den Fräsflächenmaximalradius evtl. nur umschließt oder diesen zusätzlich distal abschirmt. Ein zweiter Abschnitts kann eine mit geringerer radialen Ausdehnung gegenüber dem ersten Abschnitt aufweisen, z.B. kann dieser zweite Abschnitte die Schutzkappe umfassen, vorzugsweise ausgebildet als geschlossenes, sich distal von dem ersten Abschnitt mit dem Schutzring ersteckendes Halbkugelsegment oder auch als ringförmiger Schutzkragen, der außenseitig ebenfalls die Geometrie eines Halbkugelsegments aufweist.

Als zweckmäßig hat sich auch die diskus-förmige Ausbildung des Schutzrings erwiesen, welcher sich von dem Fräsflächenmaximalradius radial nach außen vorzugsweise kontinuierlich in der Länge verjüngt und somit einen Diskus bzw. Keil um oder distal von dem Fräsflächenmaximalradius am umfänglichen Außenrand bildet.

Als besonders vorteilhaft hat sich eine zumindest einseitig, bevorzugt beidseitig abgeschrägte und damit keilförmige Ausbildung des Schutzrings zumindest am radialen Außenrand herausgestellt, weil dieses das Wegschieben des umliegenden Gewebes ohne Verletzungen vereinfacht. Die Schräge bewirkt dabei die Zerlegung der axial entlang der Keillängsachse, also quer zur Rotationsachse des Fräser aufgebrachten Kraft in zumindest eine von der schrägen Fläche auf das Gewebe wirkende Normalkraft bzw. bei der bevorzugten Ausführungsform von beiden Keilflächen aufgebrachte Normalkräfte.

Die Abschrägung bzw. radial nach außen gehende Verjüngung kann direkt anschließend an den Fräsflächenmaximalradius beginnen der einen sich zunächst nicht verjüngenden Zwischenabschnitt zwischen dem Fräsflächenmaximalradius und dem konisch ausgebildeten bzw. sich verjüngenden radialen Außenrand des Schutzrings umfassen.

Ausführungsformen sehen einen gleiche oder unterschiedliche proximale Keilwinkel $\beta_1$ und distale Keilwinkel $\beta_2$ vor. Der proximale Keilwinkel $\beta_1$ ist der zwischen einer sich quer zur Längserstreckungsrichtung (Rotationsachse) durch die Mitte des Schutzrings erstreckende Querlinie und einer Verlängerung der proximalen Oberfläche des Schutzrings bzw. einer an dieser Oberfläche anliegenden Tangente eingeschlossene Winkel, und der distale Keilwinkel $\beta_2$, der zwischen der Querlinie und einer Verlängerung der distalen Oberfläche des Schutzrings bzw. einer an diese Oberfläche anliegenden Tangente eingeschlossene Winkel. Die proximale und distale Oberfläche des keilförmigen Schutzrings kann also flächig und gekrümmt (konvex, konkav oder als Freiformfläche) ausgebildet sein. Die Keilfläche kann sich entweder vom Fräsflächenmaximalradius bis zur vorderen Rundung erstrecken oder einen weiteren, beliebig gestalten Zwischenabschnitt umfassen.

Als besonders Vorteilhaft haben sich bei der Entwicklung der Erfindung Keilwinkel von

$$1,5° \lesssim \beta_1 \lesssim 45°$$

sowie

$$-35° \lesssim \beta_2 \lesssim 45°$$

erwiesen.

Bei der Entwicklung der Erfindung hat sich ferner herausgestellt, dass der Winkel $\beta_1$ durch die Anatomie des Patienten determiniert ist. Die Knochenoberfläche bestimmt nämlich den sinnvollen Winkel. Bei krankhafter Verengung des Wirbelkanals (Spinalkanalstenose), einer Indikation für die operative Behandlung müssen die Winkel entsprechend angepasst werden. Bei Eingriffen an der Wirbelsäule sind die anatomischen Verhältnisse und resultierenden proximalen Keilwinkel $\beta_1$ folgendermaßen:

| Operationsbereich | $\beta_1$ |
| --- | --- |
| Halswirbelsäule dorsal | 20° |
| Brustwirbelsäule dorsal | 17,5° |
| Lendenwirbelsäule dorsal (ohne Spinalkanalstenose) | 29° |
| Lendenwirbelsäule dorsal (mit Spinalkanalstenose) | 25° |

Aufgrund von leichten anatomischen Unterschieden auf jeder Höhe und der anatomischen Varietät (Normabweichungen zwischen gesunden Menschen) kann eine jeweils symmetrische Toleranz von $\pm$ 15 Grad auftreten.

Durch die Erfindung wird somit erstmalig die Möglichkeit bereitgestellt, aufgrund der Indikation des Krankheitsbildes mittels Bildgebung (z.B. Computertomografie (CT)) für jeden Patienten individuell diesen Winkel $\beta_1$ zu bestimmen und somit für den geplanten Eingriff individualisiert die "passende" Schutzanordnung einzusetzen. Dieses ist extrem nützlich, z.B. bei Deformitäten, wie Skoliosen- oder Kyphosenoperationen. Bei diesen sehr komplexen und aufwändigen, risikobehafteten Operationen ist die Osteotomie (Knochenentfernung) das geeignete Operationsverfahren. Bei diesem Operationsverfahren durchtrennt der Operateur gezielt langstreckig Knochen, um die Wirbelsäule aufzurichten (serienmäßig Laminektomien). Bei einer Pedikelsubtractionsosteotomie (geschlossene Keilosteotomie) wird hingegen der hintere Teil des Wirbels entfernt, so dass dieser nach hinten gekippt werden kann. Bei der Wirbelsäulenresektion werden durch einen hinteren Zugang zur Wirbelsäule ein oder mehrere komplette Wirbel entfernt. Dorsale Osteotomie (auch "Smith-Peterson" oder "Ponte" Osteotomie genannt) bedeutet die Entfernung der Wirbelbögen samt Facettengelenken und des interspinösen Bandapparates. Das ist auch der Fall bei der ventrodorsalen Osteotomie (von vorne und von hinten) und der Wirbelsäulenresektion (vom dorsal).

Wenn der proximale Keilwinkel $\beta1$ und der distale Keilwinkel $\beta2$ gleich sind ($\beta1=\beta2$) (symmetrische Ausführungsform) stimmt die Bezugsebene des Keilwinkels $\beta$ der Schutzanordnung mit der Ebene des Schutzanordnungsmaximalradius überein. Ein minimaler Unterschied zwischen beiden Ebenen, wenn also $\beta_1 \neq \beta_2$ hat praktisch keine Bedeutung, so dass man gleich setzen kann. Es ist also egal, ob die Bezugsebene minimal proximal von dem gesagten Schutzanordnungsmaximalradius ausgebildet ist, also $\beta_1 < \beta_2$ oder minimal distal von dem gesagten Schutzanordnungsmaximalradius, also $\beta_1 > \beta_2$. Alle drei o.g. Varianten sind zulässig und bei unterschiedlichen anatomischen Einsatzmöglichkeiten realisierbar.

Bei einer Halswirbelsäule ist es jedoch vorteilhaft, wenn $\beta1>\beta2$ ausgebildet ist, weil man somit deutlich einfacher in den engen interlaminären Spalt (zwischen beiden Wirbelbögen) eindringen kann und der epidurale Raum zwischen Kochen und Dura/Rückenmark an der HWS enger als in der BWS/LWS wird.

D.h. die Geometrie der Schutzanordnung ist für die Halswirbelsäule anders ausgebildet. Es soll $\beta1>>\beta2$ gelten bzw. die distale Auflagefläche sollte weniger konvex gestaltet sein, als bei einer Geometrie die für BWS/LWS, sollte also "flacher" ausgebildet sein.

Durch Schwenken bzw. Kippen des Schafts aus der Vertikalen kann man nunmehr also mit dem Fräser auch schräg arbeiten.

Der Kippwinkel $\kappa$ gibt die Auslenkung der Schaft-Drehachse des Fräsers von einer ideal senkrechten Vertikalen in den drei Ebenen (Koronal-, Sagital- und Transversalebene) an.

Zwei Kippwinkel in der Sagittalebene definieren den Kipp-Spielraum, welcher dem Chirurg zur Verfügung steht. Zum einen ist dieses der optimale (empfohlene) Kippwinkel $\kappa_1$, welcher bei einem dauerhaften Einsatz des Fräsers angewendet werden kann und ein größerer, ein maximal zulässiger, aber noch sicherer Kippwinkel $\kappa_2$, welcher eine kurzfristige (<2,5s) Anwendung ohne signifikante Erhöhung des Verletzungsrisikos ermöglicht.

Die Bereiche der beiden Kippwinkel $\kappa_1$ und $\kappa_2$ beziehen sich auf den proximalen Keilwinkel $\beta_1$ der proximalen Keilfläche des Schutzrings bzw. des Zwischenabschnitts und dem distalen Keilwinkel $\beta_2$ der distalen Keilfläche. Bei der Entwicklung der Erfindung hat sich gezeigt, dass dieser optimale Kippwinkel $\kappa_1$ im Bereich $|\beta_2|< \kappa_1 < -\beta_1$ liegt.

Der maximale sichere Kippwinkel $\kappa_2$ liegt im Bereich

$$|\beta_2|< \kappa_2 < -|\beta_2| \text{ , wenn } (90° - |\beta_2|) < \sigma$$

oder

$$|\beta_2|< \kappa2 < -\sigma \text{ , wenn } (90° - |\beta_2|) > \sigma.$$

Trotz dieser keilförmigen Ausbildung des Schutzrings weist der Schutzring am radialen Außenrand keinen spitzen Winkel mit spitz aufeinander zulaufenden Flächen auf, sondern ist hier abgerundet mit dem Radius "r" zur Vermeidung von Verletzungen, ist also wieder atraumatisch ausgebildet. Diese grundsätzlich keilförmige Ausbildung des Schutzrings ist also auch dann gegeben, wenn die Proximal- und Distalseite des Schutzrings nicht als gerade Flächen ausgebildet sind, sondern andere geometrische Formen aufweisen, z.B. konvex oder konkav gekrümmt sind, also z.B. einen Diskus bilden. Es handelt sich somit um eine "grundsätzlich keilförmige Gestaltung" der Proximal- und Distalseite des Schutzrings zumindest am Außenrand, so dass diese Seiten oder an diese angelegte Tangenten einen spitzen Winkel zwischen sich einschließen. Der Radius dieser äußeren Abrundung der Schutzanordnung "r" sollte in folgenden Bereichen liegen, um einfach in den engen interlaminären Spalt (zwischen beiden Wirbelbögen) von hinten oder unter die Knochenkante (z.B. bei Retrospondylophyten an der HWS) von vorne) eindringen zu können, aber die dünne Dura nicht zu verletzen (Diese weist typischerweise eine variierende Dicke von Durchschnittlich 0.307$\pm$0.122 mm auf).

| Operationsbereich | r |
|---|---|
| Halswirbelsäule von ventral | 0,15-0,50mm |
| Halswirbelsäule von dorsal | 0,20-0,75mm |
| Brustwirbelsäule von dorsal | 0,33-0,75mm |
| Lendenwirbelsäule von dorsal | 0,33-1,25mm |

Ausführungsformen der Erfindung sehen vor, dass die Schutzanordnung vibriert und auf das umliegende Gewebe überträgt, wobei die keilförmige Ausbildung des Schutzrings besonders vorteilhaft zur schonenden Auflösung des behandelten Gewebes beiträgt.

Vorzugsweise wird eine hohe Schlagfrequenz mit geringer Schlagenergie dadurch auf die keilförmige Schutzanordnung übertragen und somit das Gewebe umliegende Gewebe zweischichtig gespalten (z.B. klebende Dura, Muskel- oder Bänderansätze vom Knochenrand bzw. Knochenhaut sanft abgelöst).

Die Schutzanordnung kann Abschnitte mit mehreren Geometrien bzw. unterschiedliche Geometrieabschnitte zur Realisierung unterschiedlicher Funktionen aufweisen. Als besonders zweckmäßig hat sich die Ausbildung eines ersten Abschnitts als Schutzring erwiesen, der den Fräsflächenmaximalradius umschließt oder nach zumindest distal abschirmt und eine deutlich größere, vorzugsweise 2-fach größere radiale Ausdehnung als ein sich distal an diesen Schutzring anschließender, zweiten Abschnitt aufweist.

Die Schutzanordnung kann als getrenntes Bauteil an dem Fräskopf befestigt sein. Die eingesetzten Befestigungsmittel können z.B. einen Gewindebolzen umfassen, insbesondere mit einem Innensechskantschraubenkopf. Ein Schutzkragen umschließt somit das Befestigungsmittel vor einem Kontakt mit dem umgebenden Gewebe, so dass die Schutzanordnung also gewebeschützend für das Befestigungsmittel ausgebildet ist. Der Vorteil dieser Ausgestaltung liegt darin, dass das umgebende Gewebe durch den Schutzkragen geschont wird, der Schutzring aber gleichzeitig eingesetzt werden kann zum Eindringen in spaltförmige Engstellen und umliegendes Gewebe fernzuhalten. Die Verbindungstechnik zwischen Schutzanordnung und Fräskopf kann aber auch eine gestielte Kopplungskugel umfassen, die in eine korrespondierend ausgebildete Einsatzöffnung an dem Fügepartner rastend eingreift und insbesondere einen Hinterschnitt umfasst. Diese Ausführungsform ist besonders bei einer Schutzanordnung aus Kunststoff, insbesondere umfassend PTFE/Teflon® sinnvoll, welches die erforderliche Nachgiebigkeit zum Einrasten in den Fügepartner aufweist.

Bei einer bevorzugten Weiterentwicklung ermöglicht die Ausbildung der Fräsfläche an der proximalen Seite des Schutzrings, bevorzugt mittels Verzahnung oder Diamantbeschichtung, auch das Abfräsen an schwer zugänglichen, verdeckten Stellen von z.B. knöchernen Anbauten (z.B. von sog. Osteophyten oder Spondylophyten), wodurch ein sogenanntes "Undercutting" d.h. Unterschneiden ermöglicht wird. Dieses ist ein neues Operationsverfahren, welches bevorzugt das Abtragen von distal tiefer, auf der Rückseite liegendem Knochenteil ermöglicht, um die biomechanisch relevante äußere Knochenschicht zu erhalten. Dieses kann dank der Erfindung jetzt auch ohne Sichtkontrolle mit einem hohen Grad der Sicherheit abgefräst werden.

Die Schutzanordnung kann als Schutzaufsatz lösbar verbindbar mit dem distalen Ende des Fräskopfs ausgebildet sein, was z.B. über eine Rastverbindung erfolgt, z.B. über eine Kugelraste, oder mittels einer Schraubverbindung.

Die Schutzanordnung kann z.B. ausgebildet sein als eine distal aufsteckbare Schutzkappe, welche die Geometrie eines Kugelsegments aufweist, das einen Maximalradius aufweist, der größer ist als der Fräsflächenmaximalradius am unteren distalen Ende des Fräsers. Bevorzugt umfasst diese Schutzkappe ferner einen zumindest das untere Ende der Fräsfläche am Fräsflächenmaximalradius umfänglich einschließenden Schutzring, der geringfügig über den Fräsflächenmaximal Radialausdehnung der Fräsfläche hinausragt, diesen Bereich also ringförmig einschließt.

Die vorzugsweise kugelsegmentförmige Schutzkappe kann auf ihrer in Einbaulage oberen Seite eine einstückig angeformte Verbindungskugel evtl. mit umfassen, die in eine korrespondierend ausgebildete mittige Ausnehmung auf der Innenseite des Fräser rastend einsteckbar ist, in welche diese Verbindungskugel also einschnappbar ist. Hierzu besteht die Schutzkappe vorzugsweise aus einem thermisch verformbaren Material, wie z.B. Teflon® oder einem anderen geeigneten, und dem Fachmann bekannten biokompatiblen Kunststoff. Die Verbindungsmittel zwischen dem der Fräsfläche bzw. dem Fräskopf und der Schutzkappe können auch ausgebildet sein, um einen Relativversetz zwischen den Bauteilen zu ermöglichen.

Ein weiterer Vorteil der getrennt ausgebildeten Schutzanordnungen als Schutzaufsatz liegt darin, dass diese stillstehen kann, während sich der Fraser aufgesetzt auf diesen dreht, so dass der Schutzaufsatz als Aufsatzlager fungiert, welches die Gewebestrukturen durch die vergrößerte Auflagefläche vor Verletzungen schont.

Bestimmte Ausführungsformen weisen eine drehfeste Verbindung der Schutzanordnung mit dem Fräser auf. Die Distalauflagefläche gleitet bei der Rotation des Fräsers somit auf dem Gewebe. Dieser Aufsatzpunkt bzw. die Aufsatzfläche bildet ein Art Fixpunkt, welche distal auf dem Gewebe gleitet und ein wesentlich präziseres Fräsen ermöglicht, weil dieses den Fräser stabilisiert und ungewünschte Vibrationen vermieden werden (Vibrationsdämpfung). Zudem ermöglicht dieses eine deutlich verbesserte Handhabung (Ergonomie), weil der Fräser vom Chirurg nicht mehr hoch und beabstandet vom Gewebe gehalten werden muss, sondern man der Fräser an der distalen Kante des Knochens und dem Gewebe (leicht) abgestützt werden kann, wenn auch nur kurzzeitig.

Bei anderen Ausführungsformen ist Schutzanordnung drehbeweglich distal an dem Fräser befestigt, wozu vorzugsweise eine Lagerverbindung vorgesehen ist, z.B. ausgebildet als Gleitlager, insbesondere umfassend ein Kugelgelenk. Damit kann die Schutzanordnung sich beim Fräsen stillstehen und dient als distales Aufsatzlager auf dem Gewebe. Dieses kann somit eine leicht elastische Verbindung zwischen der Schutzanordnung und dem Gewebe realisieren. Auch bei dieser Ausgestaltung werden Vibrationen gedämpft. Wichtig ist, dass beim eventuellen Versagen der Lagerverbindung, z.B. durch Verkleben, Blutgerinnsel o.ä.) das relativbewegliche System dann die drehfeste Ausführungsform ohne ein Verletzungsrisiko darstellt.

Vibrationen oder Schwingungen des Fräsers können den Fräsprozess negativ beeinflussen oder sogar punktuell das umliegende Gewebe schädigen. Die verbreiterte Distalauflagefläche fungiert als Auflager und passiver Absorber zur Vermeidung bzw. Reduzierung derartiger Vibration und stabilisiert somit den Fräser. Da eventuell unvermeidliche Vibrationen auf elastisches Gewebe über die größere Fläche der Schutzanordnung verteilt wird, werden Verletzungen reduziert. Gleichzeitig dämpft das elastische Gewebe aber auch diese Vibrationen.

Die Schutzanordnung kann auch ein elastisches Material (vorzugsweise PTFE/Teflon®, einer biokompatiblen Silikonart oder einem anderen elastischen biokompatiblen Werkstoff) umfassen bzw. daraus bestehen, um diesen schwingunhsdämpfenden Effekt zu verstärken.

Eine zu starke Reibung sollte bei der Operation nicht überschritten werden, weil Proteine bei Temperaturen oberhalb von 42°C denaturieren. Eine thermische Isolierung wird vorrangig durch das Verhindern des Kontaktes zwischen dem heiß werdenden Fräser und dem Gewebe realisiert, insbesondere durch eine pilzförmig vergrößerte Ausgestaltung der Schutzanordnung. Ferner kann Wärme aufgrund der größeren Masse der Schutzanordnung besser abgeführt werden, so dass die Kühlung deutlich verbessert wird, was neben der rein geometrischen Ausgestaltung auch durch geeignete Werkstoffe erfolgen kann. Eine kontinuierliche Spülung des Arbeitsbereichs zur Kühlung und zum Abtransport des Knochenmehls aus den Rillen des Arbeitsteils ist aber unerlässlich.

Die Verbindungsmittel zur Verbindung des Schutzaufsatzes können bei einer weiteren Ausführungsform eine am Distalende des Fräsers ausgebildete Schutzspitze, die sich in proximaler Richtung vom Durchmesser zur Bildung eines Hinterschnitts verjüngt. In diesen Hinterschnitt ist z.B. ein umlaufender Innenrand eines torusförmigen Schutzrings einsetzbar bzw. einschnappbar.

Die Verbindungsmittel zwischen Schutzaufsatz und Fräskopf können aber auch ausgebildet sein, um eine Relativbewegung zwischen den Teilen zu ermöglichen. Vorzugsweise ist der Maximalumfang der Schutzanordnung (Schutzanordnungsmaximalumfang) etwa 1,5 bis 2 Mal so groß wie der Maximalumfang der Fräsfläche.

Die Erfindung ermöglicht damit ein Operationsverfahren unter Verwendung eines Fräsers mit einem an einem Schaft ausgebildeten Fräskopf, der eine Fräsfläche mit einem Fräsflächenmaximalradius aufweist, und ferner eine Schutzanordnung umfasst, die zumindest den Fräsflächenmaximalradius umfänglich umschließt und einen Schutzanordnungsmaximalradius aufweist. Bei dem Operationsverfahren wird der Fräser mit der Schutzanordnung in einen Spalt zwischen zwei angrenzenden Knochen, z.B. Wirbeln eingebracht, sodann der Schaft des Fräsers durch eine Antriebseinheit angetrieben und dabei wird mit der proximalen Fräsfläche Gewebe und/oder der oder die Knochen von einer Rückseite abgetragen.

Das Operationsverfahren ermöglicht z.B. eine sichere Erweiterung des interlaminären Spalts und die Einführung des Fräsköpfs direkt in die Tiefe (zum Lig. flavum und Dura) und Fräsen von innen nach außen, bzw. in der Tiefe an der Duraebene unter Erweiterung über alle 3 Knochenschichten von Wirbelbogen gleichzeitig. Dieses ermöglicht eine höhere Fräsleistung bei gleichzeitigem Schutz des umgebenden Gewebes durch die Schutzanordnung.

Beim Operationsverfahren kann die Fräsfläche mit der Schutzanordnung an der Unterkante des Knochens eingeschlossen werden kann, womit erstmalig ein Unterschneiden (Undercutting) bzw. Unterhöhlen des Knochens von innen ermöglicht wird, um biomechanisch relevanter Instabilität vorzubeugen. Dieses neuartige Operationsverfahren arbeitet insofern nach dem schrägen Einschieben des Fräskopfs in eine Öffnung von unten bzw. von innen nach außen. Man entfernt dabei nur die relevanten, einengenden unteren Knochenschichten, wobei die äußeren Knochenschichten unberührt bleiben, so dass z.B. die biomechanische Funktion einer Wirbelsäule erhalten bleibt. Dieses ist bislang nur bedingt möglich und mit mehr Verletzungsrisiken verbunden.

Insbesondere aufgrund des die Fräsfläche am Maximalradius bzw. -umfang umschließenden Schutzgürtels bzw. Schutzrings kann der Fräser angewinkelt in eine Öffnung zwischen zwei Knochen eingeschoben werden. Sodann kann der Fräser angetrieben und durch Ausüben von Zugkraft auf den Schaft das innenseitige Gewebe von dem Knochen und den Knochen selbst abgetragen werden, und zwar in jeder Winkelstellung und durch Aufbringen von allen gewünschten Kräften, weil die Schutzanordnung die umgebenden Gewebestrukturen wirksam gegen Verletzungen schützt. Dieses ist z.B. bei Operationen am Rückenmarkkanal von besonderer Bedeutung, weil bei bestehenden Operationsverfahren leicht Verletzungen auftreten. Nunmehr wird von dem Operateur wesentlich weniger Erfahrung und Sensibilität benötigt. Beispielsweise kann der Fräser in das interlaminäre Fenster zweier Wirbelbögen zwischen zwei Wirbelgelenken eingebracht werden. Da wesentlich höhere Kräfte aufgebracht werden können und die Gefahr von ungewollten Verletzungen vollständig ausgeschlossen ist, konnte mit dem Verfahren die Operationszeit an einem Wirbel von 25 Minuten auf 5 Minuten reduziert werden. Im Ergebnis kann das neue Operationsverfahren schneller, wesentlich effizienter und sicherer ausgeführt werden bei gleichzeitiger Senkung der intraoperativen Komplikationen. Dieses verwirklicht erhebliche medizinische, soziale und wirtschaftliche Vorteile. Durch die Erfindung sind somit auch roboter-gestützte Eingriffe möglich, z.B. an der Wirbelsäule, wobei man ohne Sichtkontrolle, also quasi "blind" einen rotierenden Fräskopf in einem automatischen Verfahren zur Einsatzstelle einfügt und beim Rückzug oder durch Bewegung zur Seite Knochen abfräst.

Der erfindungsgemäße Fräser kann sogar in einem automatisierten bzw. robotergestütztem Verfahren quasi "blind" eingesetzt verwendet, weil beim Einführen, Einsatz und Führen viele Ungenauigkeiten und Fehler ausgeglichen werden können.

Das Operationsverfahren verkürzt somit sowohl den stationären Aufenthalt, senkt die Anzahl und Häufigkeit der Komplikationen und Revisionsoperationen sowie eventuelle Wiederaufnahmen und reduziert die postoperative Arbeitsunfähigkeit.

Insbesondere ist das vorgeschlagene Operationsverfahren bei endoskopischen Eingriffen von Vorteil, weil man in dieser Technik grundsätzlich in einem 2-dimensionalen, "flachen" Feld (ohne Tiefenschärfe) arbeiten muss, und alle tiefer gelegenen Strukturen verschmelzen optisch mit dem Vordergrund. Die wahrscheinliche Lokalisation der nicht immer sichtbaren Strukturen ergibt sich hiermit aus den anatomischen Vorkenntnissen, der bildgebenden Diagnostik und vor allem aus der Erfahrung des Operateurs.

Das neue Verfahren kann sowohl in der Anfangsphase jedes Eingriffs an der Wirbelsäule (Hals-, Brust- und Lendenwirbelsäule) über einen dorsalen Zugang (von hinten, von der Rückenseite), als auch in einer weiteren Phase einer Operation ventral (von vorne) zumindest zweifelfrei an der Halswirbelsäule angewendet werden.

Dorsal liegen die Wirbelbögen (Lamina) zweier angrenzender abschnittsweise überlappend, ähnlich wie Dachziegel mit einem engen Spalt dazwischen übereinander.

In diesen engen Raum von kleiner 3 mm kann man ohne erhöhtes Verletzungsrisiko einen stumpfen Gegenstand einführen, weil man zwischen zwei Knochenschichten agiert. Bislang erfolgt der dorsale Zugang zum Spinalkanal mittels der "erweiterten interlaminären Fensterung". Bei diesem bekannten Operationsverfahren wird mit einem Fräser die Knochensubstanz schichtweise bis zur Dura abgetragen, um ein "Fenster" für den Zugang zu erhalten und dann unter Sichtkontrolle den eigentlichen Eingriff (z.B. Entfernung eines Bandscheibenvorfalls oder Tumors) fortführen zu können. Erweiterte Zugänge heißen Hemilaminektomie (Entfernung einer Hälfte des Wirbelbogens) oder Laminektomie (auf beiden Seiten). Wichtig ist das dieser Spalt in der Richtung des Spinalkanals am gelben Band (lig. flavum) oder übergangslos direkt an der Dura endet, was das Verletzungsrisiko extrem erhöht.

Bei dem bevorzugten Operationsverfahren wird zunächst die obere (oberflächliche) Lamina mit einer Vorschubrichtung kopfwärts abgefräst und sodann die untere (tiefere) Lamina mit einer Vorschubrichtung fußwärts entfernt, wobei die die Schutzanordnung auf oder an der Dura angeordnet ist.

Durch die erfindungsgemäß keilförmige Ausbildung des Schutzrings an dem Fräser kann nun erstmalig sanft mit und ohne Rotation des Fräsers, d.h. ausgeschaltet, in diesen engen Spalt eingeführt werden. Aufgrund des durch den Schutzring gebildeten Hebelarms kann man mit dem Schutzring den Knochenspalt zwischen den Wirbeln leicht um etwa 0,5-2 mm aufweiten. Dieses wird durch die keilförmige Ausbildung ferner erleichtert. Das Fulcrum als Angelpunkt des Hebels befindet sich auf der Kontaktfläche der gegenüberliegenden

Schutzanordnungsdistalfläche und der oberen Oberfläche des tiefer liegenden Wirbelbogens. Somit kann der Chirurg sich mit dem Fräser schichtweise in die Tiefe einarbeiten.

Mit einer drehfest befestigten keilförmigen Schutzanordnung ist sogar eine leichte Vibration bzw. Oszillation sehr vorteilhaft, weil man diese leichten Schwingungen des Fräsers durch rhythmisches Einbringen des Keils für die Erweiterung des Spalts einsetzen kann. Auf diese Weise werden z.B. Adhäsionen (Verwachsungen des Bindegewebes und Verklebungen der Dura), Ansätze der Bänder usw. sanft und sicher gelöst bzw. stumpf aufgelöst.

Bei dem Operationsverfahren können durch den Fräser abwechselnd oder gleichzeitig drei Mechanismen des Eindringens verwendet werden, nämlich der durch den Schutzring bzw. die Schutzanordnung gebildete Hebelarm, die Keilform sowie die Vibration.

Mögliche Einsatzgebiete des Fräsers und der mit diesem durchgeführten Operationsverfahren sind beispielhaft und nicht beschränkend in der nachfolgenden Tabelle dargestellt:

| Zugang / Disziplin | Eingriff bzw. direkte Einsatzstelle von neuen Fräsköpfen | Beispiele der zu Grunde liegenden Pathologien und Erkrankungen |
|---|---|---|
| Dorsaler Zugang zur Lenden- und Brustwirbelsäule - offen mikrochirurgisch und endoskopisch | Laminektomie, Hemilaminektomie, Teil-hemilaminektomie, erweiterte interlaminäre Fensterung, knöcherne Dekompression von Wirbel- und Wurzelkanal (Spinalkanals), spinale Fusion (PLIF, TLIF usw.), Laminoplastie | Spinalkanalstenose (Einengung von Spinalkanal), Bandscheibenvorfall LWS, Spondylolisthese, Traumafolgen, z.B. Frakturen, Instabilitäten, Blutungen, Tumore z.B. Ependymome, Meningeome, Neurinome, Entzündungen (Abszess, Spondylodiszitis) |
| Ventraler Zugang zur LWS und BWS offen und torakoskopisch | Osteophytenabtragung, knöcherne Dekompression, Stabilisierung, Wirbelkörperersatz-Implantation, Fusion (ALIF), Bandscheibenprothesenimplantation | Spinalkanalstenose Bandscheibenvorfall,Spondylolisthese, Traumafolgen, z.B. Frakturen, Instabilitäten, Blutungen, Tumore z.B. Ependymome, Meningeome, Neurinome, Entzündungen (Abszess, Spondylodiszitis) |
| Dorsaler Zugang zur Halswirbelsä ule | Operation nach Frykholm, Laminektomie, Hemilaminektomie, Laminoplastie, knöcherne Dekompression des Spinalkanals | Spinalkanalstenose Bandscheibenvorfall,Spondylolisthese, Traumafolgen, z.B. Frakturen, Facettengelenks(sub)lux ationen Instabilitäten, Blutungen, Tumore z.B. Ependymome, Meningeome, Neurinome, Entzündungen (Abszess, Spondylodiszitis) |
| Ventraler Zugang zur HWS | Mikrodiskektomie und Fusion (ACDF), Uncoforaminotomie, Ventrale knöcherne Dekompresion, Osteophytenabtragung | Spinalkanalstenose Bandscheibenvorfall, Blutungen, Tumore z.B. Ependymome, Meningeome, Neurinome, Entzündungen (Abszess, Spondylodiszitis) |
| Transnasaltranssphenoi daler Zugang mikrochirurg isch und endoskopisch | Erweiterung des Fensters vom Keilbeinhöhle, Zugang zur Sella turcica | Hypophysenprozeße z.B. Adenome |
| Retrosigmoidaler und subtemporaler Zugang | Partielle Petrosektomie, Erweiterung von inneren Gehörgang | Akustikusneurinome, Menigeome |
| Schädelbasis Chirurgie, diverse Zugänge | Preparation diverser Nerven und Gefäße an der Schädelbasis, im Foramen magnum, petroclival, Foramen jugulare, Cavum Meckeli | Diverse Prozesse |
| Mund-Kiefer-Gesichtschirurgie | Sinus lift | Vorbereitungsmaßnahme für Zahnimplantate |
| Mund-Kiefer-Gesichts-chirurgie | Präparation und Transposition von Nervus alveolaris inferior im Canalis mandibulae ab Foramen mentale | Vorbereitungsmaßnahme für Zahnimplantate |
| Endoskopische Kiefer- und Stirnhöhlenc hirurgie | Diverse schleimhaut- und gefässschonende Eingriffe | Tauchrohr-OP |
| Minimalinvasive Fußchirurgie | Schneiden/Verkürzen von Knochen über eine punktuelle Inzision | z.B. Halux valgus-OPs |
| Arthroskopie diverser Gelenke | Osteophytenabtragung | |

Die Schutzanordnung kann somit angepasst an die vorliegenden anatomischen Verhältnisse bedarfsgerecht z.B. unterschiedliche Konvexitäten aufweisen und so den Platz bzw. den o.g. Sicherheitsabstand zu den Weichteilen bestimmen.

Im Rahmen der Erfindung sind viele weitere Ausgestaltungen der Schutzanordnungen des Fräsers, z.B. dessen Schutzgürtel und Schutzkappe möglich, so z.B.

- unterschiedliche geometrische Ausgestaltungen des Fräskopfes, so z.B. auch variable konvexe, gerade oder konkave Unterformen,

- diverse Ausführungen der Verzahnung oder Diamantbeschichtung der scharfen Fräsfläche,

- das Ausmaß der Ausbreitung der scharfen Fräsfläche auf den proximalen, radialen Rand des Schutzrings,

- unterschiedliche geometrische Formen im Sinne von Größe, Dicke, Länge bzw. Umfang als auch Winkelgrad der Konvexität - unabhängig den Schutzgürtel bzw. Schutzring als auch die glatte Schutzkappe betreffend

Die geometrische Ausgestaltung des Fräskopfes bestimmt den möglichen, sicheren Kippwinkel beim Fräsen. Der Operateur wählt somit abhängig von den anatomischen Verhältnissen und dem gewünschten Endergebnis ein geeignetes und für den Anwendungsfall passenden Fräser mit Schutzanordnung aus.

Die distale Schutzanordnung, die vorzugsweise in einen konzentrischen überragenden Schutzkragen übergeht, ist vorzugsweise so ausgebildet, dass diese reibungslos über alle darunter und daneben befindliche Strukturen gleiten und gleichzeitig diese sensiblen Strukturen sanft von den scharfen Schneiden/scharfen Reibfläche des Arbeitsteils fernhalten.

In der folgenden ausführlichen Beschreibung wird auf die beigefügten Zeichnungen Bezug genommen, die Teil dieser Erfindungsbeschreibung bilden und in denen zur Veranschaulichung spezifischer Ausführungsformen gezeigt sind, mit denen die Erfindung ausgeübt werden kann. In dieser Hinsicht wird Richtungsterminologie wie etwa "oben", "unten", "vorne", "hinten", "vorderes", "hinteres", usw. in Bezug auf die Orientierungen der beschriebenen Figur(en) verwendet. Da Komponenten von Ausführungsformen in einer Anzahl verschiedener Orientierung positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Die folgende ausführliche Beschreibung ist nicht im einschränkenden Sinne aufzufassen.

Im Rahmen dieser Beschreibung werden die Begriffe "verbunden", "angeschlossen" sowie "integriert" verwendet zum Beschreiben sowohl einer direkten als auch einer indirekten Verbindung, eines direkten oder indirekten Anschlusses sowie einer direkten oder indirekten Integration. In den Figuren werden identische oder ähnliche Elemente mit identischem Bezugszeichen versehen, soweit dieses zweckmäßig ist.

Bezugszeichenlinien sind Linien, die das Bezugszeichen mit dem betreffenden Teil verbinden. Ein Pfeil hingegen, der kein Teil berührt, bezieht sich auf eine gesamte Einheit, auf die er gerichtet ist. Die Figuren sind im Übrigen nicht unbedingt maßstäblich. Zur Veranschaulichung von Details können möglichweise bestimmte Bereiche übertrieben groß dargestellt sein. Darüber hinaus können die Zeichnungen plakativ vereinfacht sein und enthalten nicht jedes bei der praktischen Ausführung gegebenenfalls vorhandene Detail. Die Begriffe "oben" und "unten" beziehen sich auf die Darstellung in den Figuren. Es zeigen:

Figur 1 eine Seitenansicht einer ersten Ausführungsform eines erfindungsgemäßen Fräsers,

Figur 2 eine Draufsicht des Fräsers gemäß Figur 1;

Figur 3 einen Querschnitt des Fräsers gemäß Figur 1,

Figur 4 eine vergrößerte perspektivische Darstellung des Fräskopfes des Fräsers gemäß Figur 1;

Figur 5 eine Seitenansicht einer zweiten Ausführungsform eines erfindungsgemäßen Fräsers;

Figur 6 eine Draufsicht des Fräsers gemäß Figur 5;

Figur 7 einen Querschnitt entlang der Linie B-B gemäß Figur 6;

Figur 8 eine vergrößerte perspektivische Darstellung des Fräskopfes der zweiten Ausführungsform;

Figur 9 die durch die erfindungsgemäße Ausbildung des Fräskopfes gebildete Schutzzone, die beim Fräsen das benachbarte vulnerable Gewebe umgibt;

Figur 10 eine vergrößerte isometrische Draufsicht des Fräskopfs einer dritten Ausführungsform mit abgenommener Schutzkappe;

Figur 11 eine isometrische Seitenansicht der dritten Ausführungsform von unten;

Figur 12 eine isometrische Seitenansicht der dritten Ausführungsform;

Figur 13 eine verkürzte Seitenansicht der dritten Ausführungsform;

Figur 14 einen vergrößerten Querschnitt des Fräskopfes der dritten Ausführungsform;

Figur 15 eine vergrößerte isometrische Draufsicht des Fräskopfes einer vierten Ausführungsform mit abgenommenem Schutzring;

Figur 16 eine perspektivische Ansicht der vierten Ausführungsform von unten;

Figur 17 einen vergrößerten Querschnitt des Fräskopfs der vierten Ausführungsform mit aufgesetztem Schutzring;

Figur 18 eine Seitenansicht der vierten Ausführungsform;

Figur 19 eine vergrößerte isometrische Draufsicht einer fünften Ausführungsform bei abgenommener Schutzkappe;

Figur 20 eine vergrößerte isometrische Ansicht der fünften Ausführungsform bei entfernter Schutzkappe von unten;

Figur 21 ein vergrößertes seitliches Schnittbild der fünften Ausführungsform mit eingesetzter Schutzkappe;

Figur 22 eine verkürzte Seitenansicht der fünften Ausführungsform;

Figur 23 eine isometrische Seitenansicht der fünften Ausführungsform;

Figur 24 eine Seitenansicht einer sechsten Ausführungsform mit sphärischem Fräskopfes und Schutzgürtel;

Figur 25 eine Seitenansicht einer siebten Ausführungsform eines Fräskopfes mit abgeflachter Schutzkappe und Schutzgürtel;

Figur 26 eine vergrößerte isometrische Seitenansicht eines Fräskopfes einer achten Ausführungsform bei abgenommener Schutzkappe;

Figur 27 eine vergrößerte isometrische Ansicht des Fräskopfes der achten Ausführungsform von unten;

Figur 28 eine andere vergrößerte isometrische Draufsicht der achten Ausführungsform mit aufgesetzter Schutzkappe;

Figur 29 einen vergrößerter Querschnitt des Fräskopfes der achten Ausführungsform bei zentrierter Ausrichtung der Schutzkappe im Verhältnis zum Fräskopf;

Figur 30 ein verkleinerter Querschnitt des Fräskopfes der der achten Ausführungsform mit der Schutzkappe in versetzter Ausrichtung im Verhältnis zum Fräskopf;

Figur 31 eine Seitenansicht der achten Ausführungsform

Figur 32 eine vergrößerte Seitenansicht eines Fräskopfes einer neunten Ausführungsform mit einer flachen Oberseite des Schutzrings;

Figur 33 eine vergrößerte Seitenansicht eines Fräskopfes einer zehnten Ausführungsform mit einem Diskusförmigen Schutzring mit gleicher Wölbung auf Ober- und Unterseite;

Figur 34 eine vergrößerte Seitenansicht eines Fräskopfes einer elften Ausführungsform mit einem Schutzring mit nur leicht gewölbter Unterseite und stärker gewölbter Oberseite;

Figur 34a eine isometrische Draufsicht der neunten, zehnten und elften Ausführungsformen gemäß Figuren 32 bis 34;

Figur 35 eine isometrische Ansicht von unten einer zwölften Ausführungsform mit einem Gewindezapfen am distalen Ende der Fräsfläche, dass in eine zentral in einem Schutzring angeordnete Gewindeöffnung eindrehbar ist;

Figur 36 eine isometrischer Querschnitt der elften Ausführungsform gemäß Figur 35;

Figur 37 eine isometrische Draufsicht einer dreizehnten Ausführungsform mit einem distal von der Fräsfläche abstehenden Gewindezapfen, auf den eine als Schutzkappe ausgebildete Schutzanordnung aufschraubbar ist;

Figur 38 eine Seitenansicht der dreizehnten Ausführungsform gemäß Figur 37 mit entfernter Schutzkappe;

Figur 39 eine isometrische Draufsicht einer vierzehnten Ausführungsform mit einer als Schutzkugel ausgebildeten Schutzanordnung mit oberseitig abgeflachter Anlagefläche, die auf den distal von der Fräsfläche abstehenden Gewindezapfen des Fräsers aufschraubbar ist;

Figur 40 eine Seitenansicht der vierzehnten Ausführungsform gemäß Figur 39;

Figur 41 ein isometrischer Längsschnitt einer fünfzehnten Ausführungsform mit frontseitig geschlossener Schutzkappe, die mit einem zentralen Gewindeloch auf den distal von der Fräsfläche abstehenden Gewindezapfen aufschraubbar ist;

Figur 42 ein isometrischer Längsschnitt einer sechzehnten Ausführungsform mit einer als Schutzring ausgebildeten Schutzanordnungen, die vorzugsweise aus Keramik besteht und über eine Mutter an dem distal von der Fräsfläche abstehenden Gewindezapfen befestigbar ist, zu deren Aufnahme eine als Sitz für die Mutter fungierende Mutteraufnahme am distalen unteren Ende vorgesehen ist;

Figur 43 ein isometrischer Längsschnitt einer siebzehnten Ausführungsform mit einer scheibenförmigen Schutzanordnung mit einem Gewindezapfen auf der proximalen Oberseite, der in ein Gewindeloch am Distalende des Fräsers eindrehbar ist;

Figur 44 ein Seitenansicht einer achtzehnten Ausführungsform mit in das Gewindeloch an dem Fräskopf eingeschraubter Schutzanordnung mit leicht konvex gewölbter Distalauflagefläche;

Figur 45 ein isometrischer Längsschnitt einer neunzehnten Ausführungsform mit einer scheibenförmigen Schutzanordnung und konkav gewölbter distaler Schutzfläche, die mittels eines proximalen Gewindezapfens in das Gewindeloch am Distalende des Fräsers eingeschraubt ist;

Figur 46 ein isometrischer Längsschnitt einer zwanzigsten Ausführungsform mit einem als Schutzring ausgebildeten Schutzanordnung, der mittels eines Gewindebolzens, der in Einbaulage in einer distalen Aufnahmeöffnung des Schutzrings aufgenommen ist, an dem Distalende des Fräskopfs festschraubbar ist;

Figur 47 eine vergrößerte Seitenansicht einer einundzwanzigsten Ausführungsform mit distal von dem Fräsflächenmaximalradius und diesen radial überragenden Schutzring, der auf einem entsprechenden Sitz am Distalende des Fräsers aufgeschnappt ist;

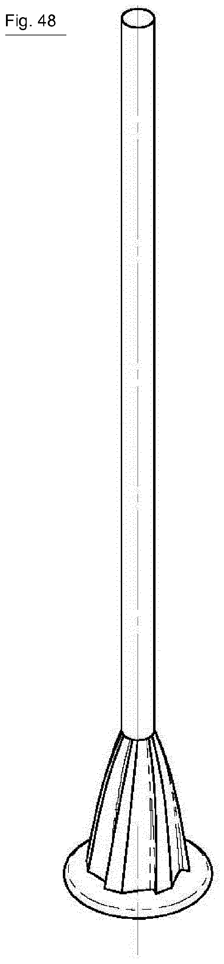

Figur 48 eine isometrische Draufsicht einer zweiundzwanzigsten Ausführungsform mit einem Schutzring, der auf das Distalende des Fräsers distal von dem Fräsflächenmaximalradius auf einen Sitz aufgeschnappt ist;

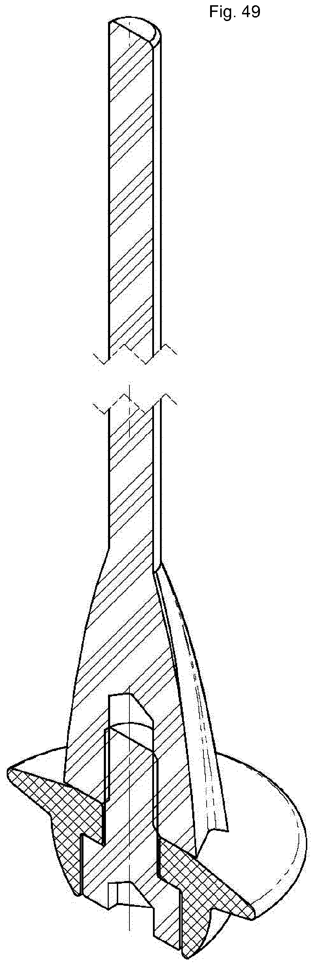

Figur 49 einen isometrischen Längsschnitt einer dreiundzwanzigsten Ausführungsform mit einem distal an der Fräsfläche über einen Befestigungsbolzen mit einem Innensechskant befestigten Schutzaufsatz, der einen sich an den Fräsflächenmaximalradius der Fräsfläche anschließenden, ersten diskusförmigen Abschnitt aufweist, der einen Schutzring um den Fräsflächenmaximalradius unter Verdopplung von dessen Außenradius bildet und an welchen sich distal erstreckend ein an der außen Seite bogenförmig ausgebildeter Schutzkragen zur mittigen Aufnahme des Befestigungsbolzen anschließt, der eine radiale Ausdehnung von etwa dem Fräsflächenmaximalradius aufweist;

Figur 50 eine Längsschnitt eines erfindungsgemäßen Fräsers zur Verdeutlichung der verwendeten Begriffe und Winkel;.

Figuren 51 bis 55 Seitenansichten der Verwendung eines erfindungsgemäßen Fräsers in einem Operationsverfahren an zwei Halswirbelbögen (Lamina), z.B. eine FrykholmOP, interlaminäre Fensterung, Hemi-, Laminektomie.

Alle Ausführungsbeispiele sind rotationssymmetrisch zur Mittelachse des Fräsers ausgebildet.

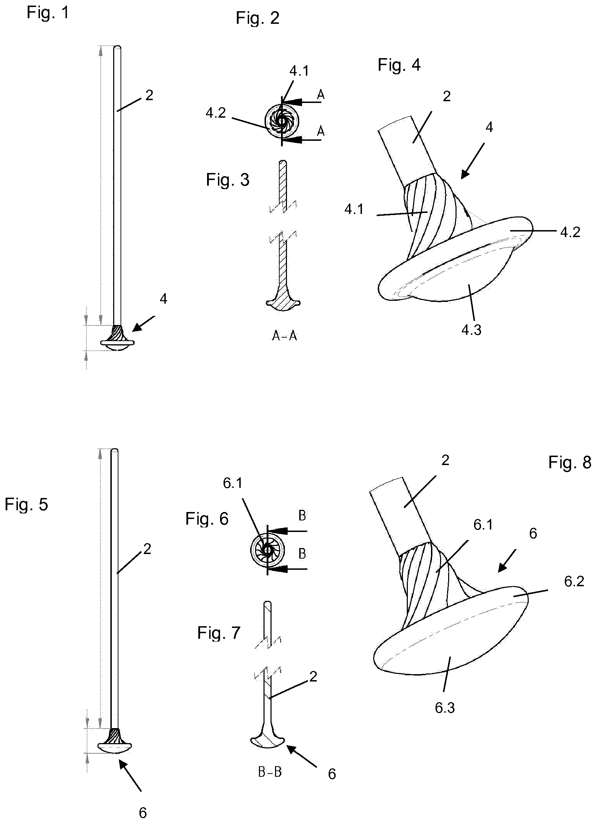

Der in Figur 1 dargestellte Fräser besteht demnach hauptsächlich aus dem Schaft 2 mit einem oberseitigen proximalen Ende zum drehfesten Verbinden mit einem bekannten Antriebsgerät und einem komplexen Fräskopf 4 am vorderseitigen Distalende, welcher eine komplexe Geometrie aufweist.

Der Fräskopf 4 umfasst eine proximal ausgerichtete bzw. wirkende, scharfe Fräsfläche 4.1 (hier mit Verzahnung), welche sich von der der äußeren Umfangsfläche des Schafts 2 in distaler Richtung verbreitert bis zu einem Maximalumfang, wo ein diesen Maximalumfang kreisringförmig umschließender Schutzring 4.2 ausgebildet ist, der deutlich radial über den Maximalumfang nach außen ragt. An dem distalen Vorderende ist mit etwas reduziertem Radius eine gegenüber dem Schutzring 4.2 zurückspringende bzw. zurückversetzte, distale konvexe Schutzkappe 4.3 ausgebildet.

Deutlich erkennbar erstreckt sich die Fräsfläche 4.1 nur bis zum Maximalumfang und geht dann in den abgerundeten Schutzring 4.2 über, der diesen Maximalumfang kreisringförmig als radialer Ring umschließt. Dieser Schutzring 4.2 kann bei der Operation verletzungsfrei in das Gewebe eingeschoben werden, und zwar auch bei rotierendem Fräser, weil der Schutzring 4.2 wie alle übrigen Flächen außer der Fräsfläche kantenfrei und abgerundet ist.

Wie die in den Figuren 1 bis 4 dargestellte erste Ausführungsform ist auch die in Figuren 5 bis 8 dargestellte zweite Ausführungsform des Fräsers mit einem alternativ ausgebildeten Fräskopf 6 versehen, wobei aber auch dieser Fräser als einstückiges Drehteil ausgebildet ist. Bei dieser zweiten Ausführungsform ist die Schutzkappe 6.3 jedoch konvex pilzförmig ausgebildet, im Gegensatz zur ersten Ausführungsform ist dies größer ausgebildet und erstreckt sich durchgängig ohne Rücksprung vom Außenrand des Schutzrings 6.2, womit die Schutzkappe 6.3 und der Schutzring 6.2 also ein durchgängiges Kugelsegment mit einer größeren Distalauflagefläche gegenüber der ersten Ausführungsform bilden.

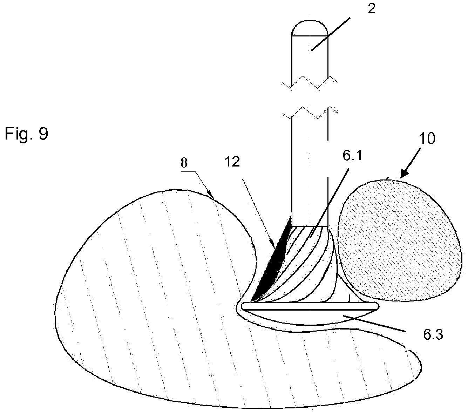

Figur 9 zeigt eine vergrößerte Seitenansicht eines Fräsers bei einer Operation eingebracht zwischen schematisch angedeuteten Gewebe 8 und Knochenstrukturen 10 zur Verdeutlichung der dreidimensionalen Schutzzone 12, welche durch den Schutzring 6.2 der Schutzanordnung gebildet wird. Diese dreidimensionale Schutzzone 12 ist in schwarz eingezeichnet und wird radial außenseitig begrenzt durch die Verbindungslinie vom Außenrand des Schutzrings 6.2 bzw. der Schutzkappe 6.3 zum proximalen Ende der Fräsfläche 6.1 und innenseitig durch die Fräsfläche 6.1 selbst. Der Rotationskörper dieses Bereichs bildet die dreidimensionale Schutzzone 8 um die Fräsfläche 6.1 herum, zu dem umgebenden Gewebe bzw. den Knochen 10, in den kein Gewebe 8 eintritt. Oder anders ausgedrückt: Die Schutzanordnung ist so ausgebildet, dass diese Schutzzone 12 während der Operation stets frei von Gewebe 8 ist. Je weiter der Schutzring 6.2 bzw. die Schutzkappe 6.3 über die Fräsfläche 6.1 hinausragt bzw. je größer deren Maximaldurchmesser ist, desto weiter wird der Maximalumfang der Fräsfläche 6.1 umspannt und desto größer ist die um die Fräsfläche 6.1 gebildete Schutzzone.

Deutlich erkennbar ist, wie die Schutzkappe 6.3 auf das Gewebe aufsetzbar ist und somit eine Verbreiterung bei Ausübung von Druck die Flächenpressung auf das Gewebe so reduziert wird, der Fräser aber gleichzeitig mit seiner Fräsfläche 6.1 ausschließlich proximal arbeitend eingesetzt werden kann, um den angrenzenden Knochen 10 proximal abzutragen.

Die durch die geometrische Gestaltung erzeugte Schutzfunktion ist in Figur 9 gut ersichtlich. Um von den abzufräsenden Knochenteile 10 die taktilen Weichteile schonend zu trennen bzw. abzuschieben und gleichzeitig gute Anbindung zum Knochen zu gewährleisten, kann die Geometrie des Fräskopfes pilzförmig gemäß der zweiten Ausführungsform gestaltet sein, wobei die distale Fläche der Schutzkappe 6.3 und des Schutzrings 6.2 glatt poliert oder so beschichtet ist, dass aufgrund des Schutzrings 6.2 alle Kanten abgerundet sind und die scharfe Arbeitsfläche proximal zum lateralen Rand des Schutzrings 6.2 vor dem Bereich der Maximalradialausdehnung endet.

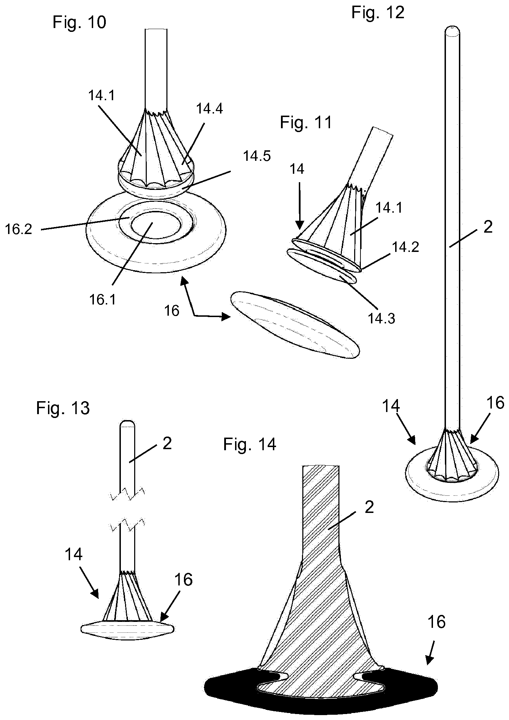

Figuren 10 bis 14 zeigen verschiedene Ansichten einer dritten Ausführungsform eines Fräsers, bei dem der Fräskopf 14 wieder einstückig am unteren Ende des Schafts 2 ausgebildet ist und sich unter Bildung der Fräsfläche 14.1 von der äußeren Mantelfläche des Schafts 2 bis zu einem unteren Maximalumfang kontinuierlich verbreiternd erstreckt, dort in eine obere Kreisplatte 14.2 übergeht, von der distal beabstandet über einen verjüngten Hinterschnitt eine untere Kreisplatte 14.3 vorgesehen ist, so dass zwischen den Kreisplatten 14.2 und 14.3 eine umlaufende Nut ausgebildet ist. In diese Nut ist die aus Kunststoff mit einer PTFE/Teflon®-Beschichtung bestehende und torusförmig ausgebildete Schutzkappe lösbar 16 einsetzbar, die auf der proximalen und der distalen Seite konvex gewölbt ist wie eine fliegende Untertasse. Der Außenradius der Schutzkappe 16 ist etwa doppelt so groß wie der Maximalradius der Fräsfläche 14.1 am Distalende. Auf der Oberseite weist diese Schutzkappe 16 eine korrespondierend ausgebildete Einstecköffnung 16.1 mit umlaufendem und nach innen ragenden, elastischen Rand 16.2 auf, so dass die unteren Kreisplatte 14.5 in die Einstecköffnung 16.1 einsetzbar und der Rand 16.2 in Einbaulage in die umlaufende Nut des Fräskopfes 14 eingreift und so die Schutzkappe 16 unverlierbar aber lösbar an dem Fräskopf 14 verbindet, wobei bei dieser Ausführungsform das Distalende der Schutzkappe 16 die Auflagefläche bildet.

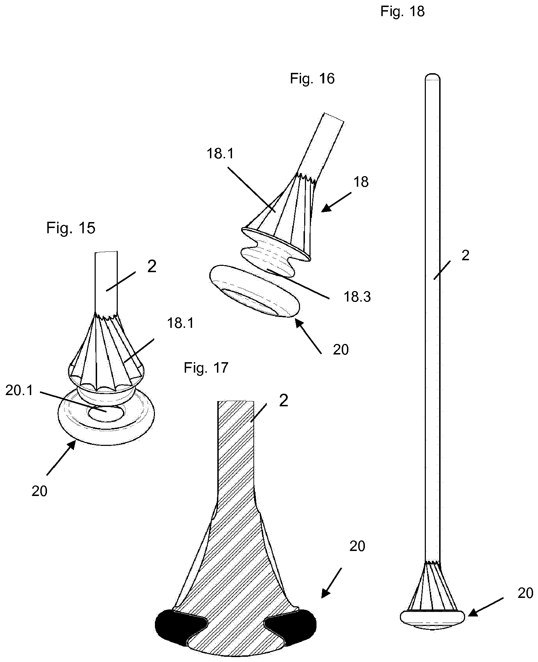

Bei der in den Figuren 16 bis 18 dargestellten fünften Ausführungsform ist die Fräsfläche 18.1 des Fräskopfes 18 wie bei der vierten Ausführungsform ausgebildet, allerdings ist das distal von der Fräsfläche 18.1 ausgebildete Befestigungsende etwas anders gestaltet, nämlich mit einer stärker konvex gewölbten Distalauflagefläche 18.3 und einem sich proximal daran anschließenden und gegenüber dieser verjüngten Hinterschnitt zur Bildung des Sitzes für den aufschnappbaren Schutzring 20, welcher bei dieser fünften Ausführungsform somit zusammen mit der Distalauflagefläche 18.3 die Schutzanordnung bildet. Dieser Schutzring 20 ist seinerseits als torusförmig geschlossener Ringkörper mit einer mittigen Einstecköffnung 20.1 ausgebildet, deren Innendurchmesser kleiner ist als der Maximaldurchmesser der einstückig distal unterhalb der Fräsfläche 18.1 an dem Fräser angeformten Distalauflagefläche 18.3. Durch diese Ausgestaltung ist der elastische Schutzring 20 unverlierbar, aber lösbar auf durch den Hinterschnitt gebildeten Sitz des Fräskopfes 18 aufschnappbar. Bei dieser Ausführungsform bildet die Auflagefläche 18.3 mit dem aufgesetzten Schutzring 20 die Distalauflagefläche der Schutzanordnung.

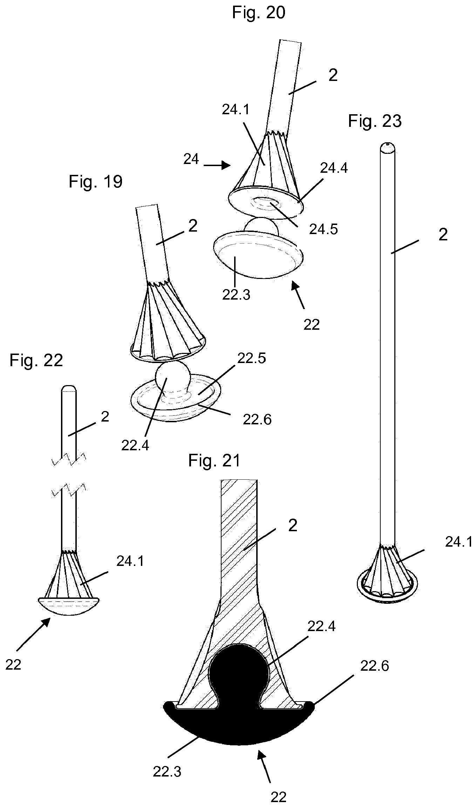

Die Figuren 19 bis 23 zeigen verschiedene Ansichten der sechsten Ausführungsform mit einer Schutzanordnung ausgebildet als Schutzkappe 22, bei welcher die Distalauflagefläche als Kugelkappe 22.3 gestaltet ist. Am Proximalende weist die Schutzkappe 22 eine einstückig angeformte, gestielte Kopplungskugel 22.4 auf, welche in eine korrespondierend ausgebildete Einstecköffnung 24.5 am distalen Vorderende des Fräskopfs 24 rastend und unverlierbar, aber lösbar einsteckbar ist. Der Fräskopf 24 weist am Distalende der Fräsfläche 24.1 eine Kreisplatte 24.4 auf, welche den Fräsflächenmaximalradius der Fräsfläche 24.1 bildet. In Einbaulage greift diese Kreisplatte 24.4 in einen korrespondierend ausgebildeten, kreisringförmigen Sitz 22.5 auf der proximalen der Schutzkappe 22 ein. An der Schutzkappe 22 ist radial außenseitig diesen Sitz 22.5 ringförmig umschließend ein sich Schutzring 22.6 vorgesehen, welcher in proximaler Richtung über die Kreisplatte 24.4 hinausragt und diese außenseitig somit in Einbaulage einfasst.

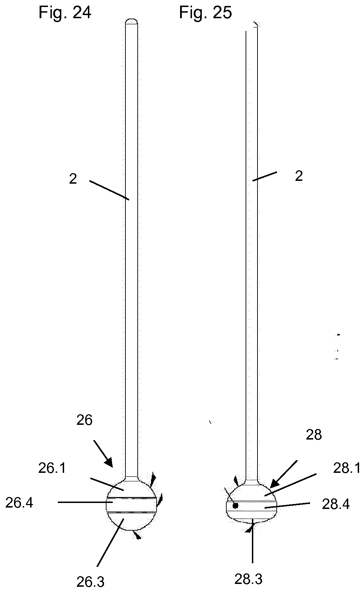

Die Figuren 24 und 25 zeigen die siebte und achte Ausführungsformen des atraumatischen Fräsers, deren Fräsflächen 26.1; 28.1 jeweils sphärisch ausgebildet sind.

Bei der siebten Ausführungsform gemäß Figur 24 sind Fräsfläche 26.1 und Schutzkappe 26.3 beide sphärisch ausgebildet, wobei die proximale Fräsfläche 26.1 mit Diamanten beschichtet ist und die distale Schutzkappe 26.3 sich über den Maximalradius proximal nach oben erstreckt zur Bildung eines umfänglich umschließenden Schutzgürtels 26.4.

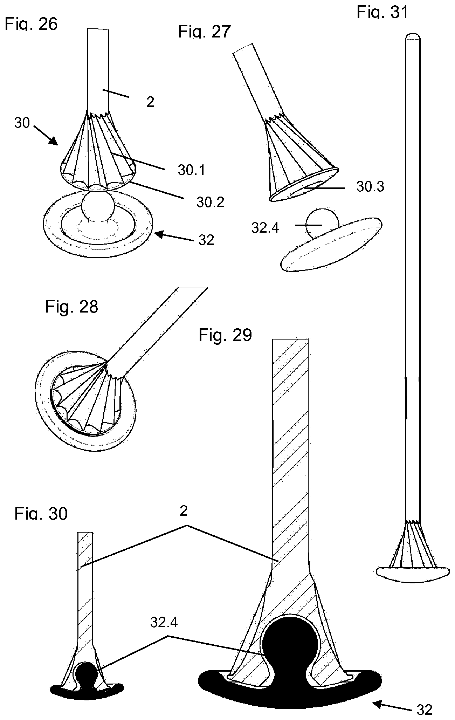

Auch bei der in Figur 25 dargestellten achten Ausführungsform ist der Fräskopf 28 zumindest auf der proximalen Fräsfläche 28.1 sphärisch ausgebildet und mit Diamanten besetzt. Die Distalauflagefläche 28.3 ist jedoch gegenüber der siebten Ausführungsform abgeflacht, weist also einen deutlich größeren Radius als die proximale Fräsfläche 28.1 des Fräskopfs 28 auf, ist aber auch als glatten Schutzkappe 28.3 ausgebildet, welche das Distalende umfänglich vollständig einfasst und sich proximal über den Maximalradius des Fräskopfes 28 hinaus wieder zur Bildung eines Schutzgürtels 28.4 erstreckt. Die in den Figuren 26 bis 31 dargestellte neunte Ausführung unterscheidet sich von der in den Figuren 20 bis 23 dargestellten fünften Ausführungsform hauptsächlich durch die Verbindungstechnik zwischen der als Schutzkappe 32 ausgebildeten Schutzanordnung und dem Fräskopf 30. An dem Distalende des Fräskopfes 30 ist distal von dem Fräsflächenmaximalradius 30.2 der Fräsfläche 30.1 eine Einstecköffnung 30.3 ausgebildet, in die eine gestielte Kopplungskugel 32.4 lösbar einsteckbar ist, also über einen Stiel an der Proximalen Oberfläche der Schutzkappe 32 einstückig angeformte Kopplungskugel 32.4. Ein gewisses Übermaß der Einstecköffnung 30.3 gegenüber dem Außendurchmesser der Kopplungskugel 32.4 erlaubt einen Relativversatz zwischen der Schutzkappe 32 und dem Fräskopf 30, vorzugsweise im Bereich von 0,5 bis 1 mm. Dieser Relativersatz ermöglicht eine mögliche Abweichung der Koaxialität der Schutzanordnung von der Rotationsachse des Fräser und realisiert somit eine verbesserte Anpassungsfähigkeit, insbesondere in engen anatomischen Verhältnissen.

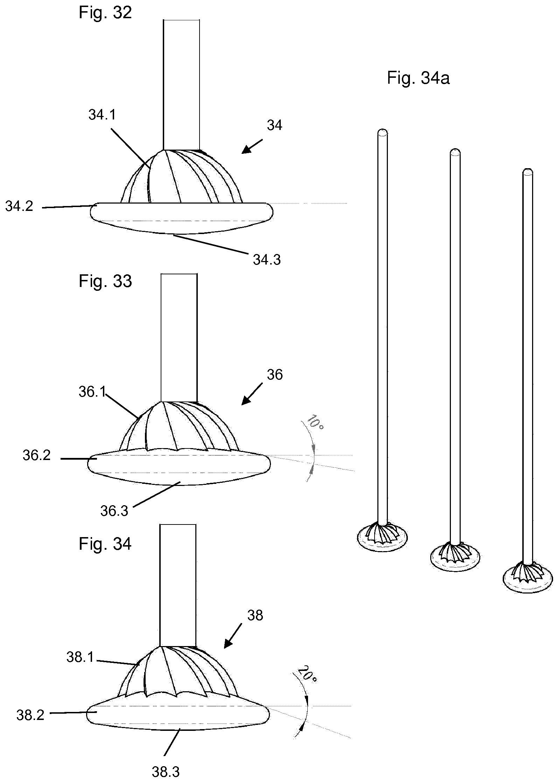

Die Figuren 32 bis 34 zeigen dann verschiedene Ausführungsformen von Fräsköpfen 34, 36 ,38 mit unterschiedlich ausgebildeten, einstückig angeformten Schutzringen 34.2; 36.2; 38.2 und Distalauflageflächen 34.3; 36.3; 38.3, die radial über den jeweiligen Fräsflächenmaximalradius der distalen Enden der Fräsflächen 34.1; 36.1; 38.1 hinausragen. Die Unterschiede betreffen hier im Wesentlichen die geometrische Ausgestaltung der Schutzanordnungen, insbesondere die proximalen Oberseiten der tellerförmig die Fräsflächen 34.1; 36.1; 38.1 umschließenden Schutzringe 34.2; 36.2; 38.2.

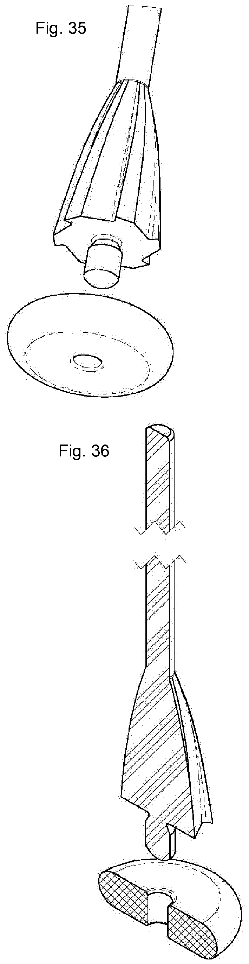

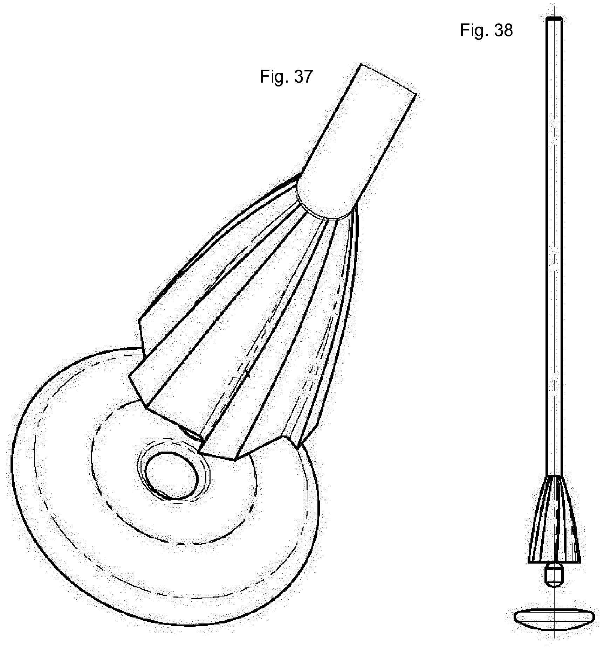

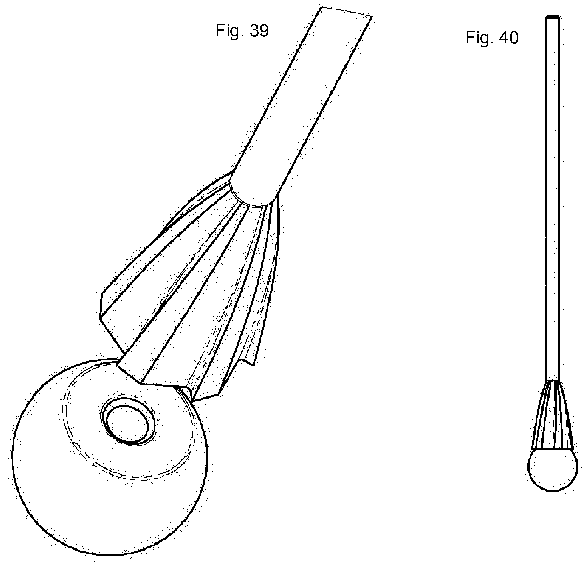

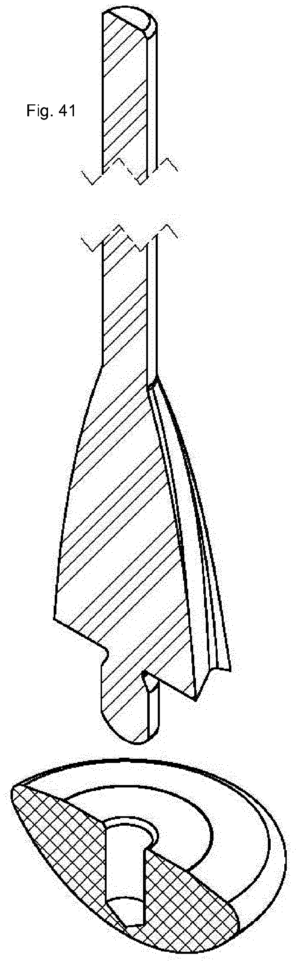

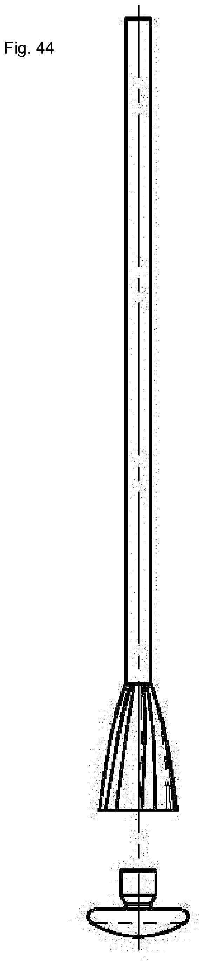

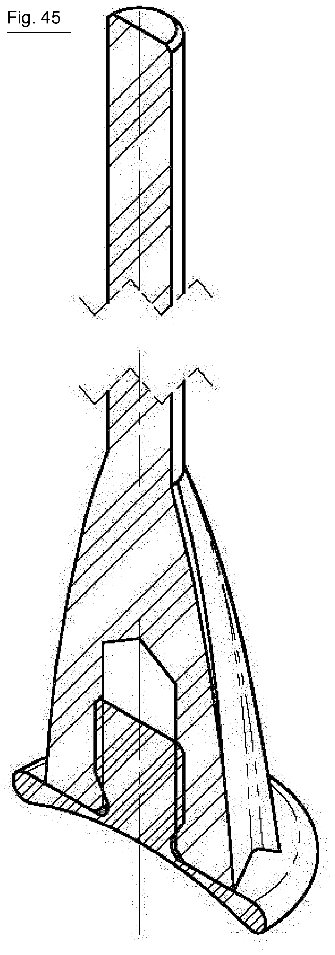

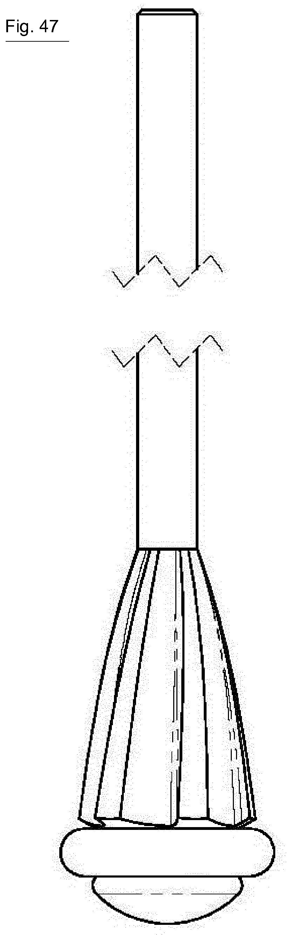

Die Ausführungsform gemäß den Figuren 35 bis 47 stellen verschiedene Ausführungsformen dar, bei welchen die Verbindungsmittel zwischen Fräskopf und der Schutzanordnung als Schraubverbindung ausgebildet sind.

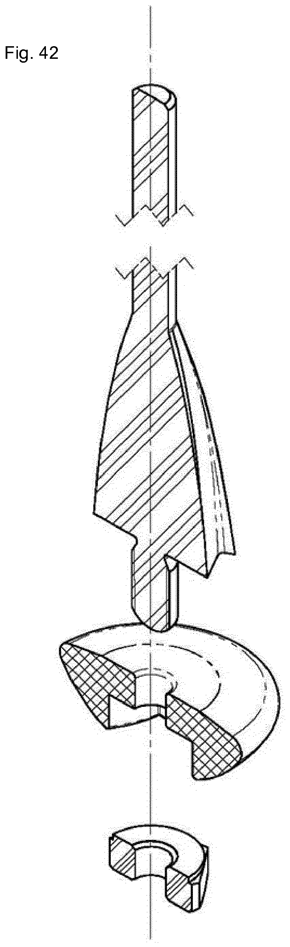

Die Ausführungsformen gemäß den Figuren 35 bis 42 sind als "männliche" Schraubverbindungen ausgebildet, bei denen an dem, in den Figuren unteren Distalende des Fräskopfes ein entlang der mittleren Rotationssachse abragender Gewindestift vorgesehen ist, auf den entweder die Schutzanordnung direkt mit einem komplementär ausgebildeten Innengewinde aufschraubbar ist oder gemäß der Ausführungsform in Figur 42 am distalen unteren Ende des ringförmigen Schutzrings eine Mutteraufnahme ausgebildet ist, also ein Komplementär zur Mutter ausgebildete Aufnahmeöffnung, in welche die Mutter drehfest sitzt und gleichzeitig durch die umgebende Schutzanordnung von dem umliegenden Gewebe vor unbeabsichtigten Verletzungen abgeschirmt wird.

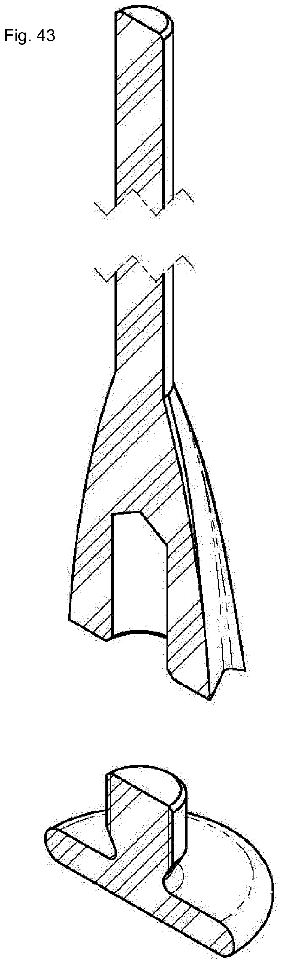

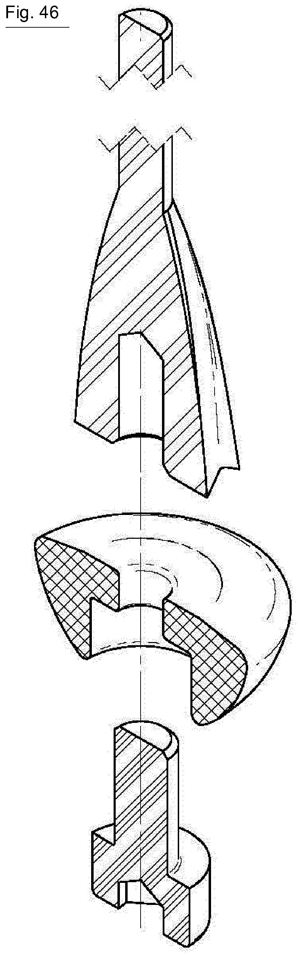

Die in den Figuren 43 bis 46 dargestellten Ausführungsformen stellen hingegen die "weibliche" Ausführungsform der Schraubverbindungen dar, bei denen an den Schutzanordnungen an den in Einbaulage proximalen Oberseiten jeweils ein Gewindestift oder Gewindezapfen vorgesehen ist, der in eine komplementär ausgebildete Gewindeöffnung an der Innenseite des distalen unteren Endes des Fräskopfs eindrehbar ist. Die Ausführungsform gemäß Figur 46 ist insofern besonders, weil bei dieser die Verbindung über eine getrennte Mutter erfolgt, welche umschlossen in einer distalen Bolzenaufnahme der Schutzanordnung sitzt.

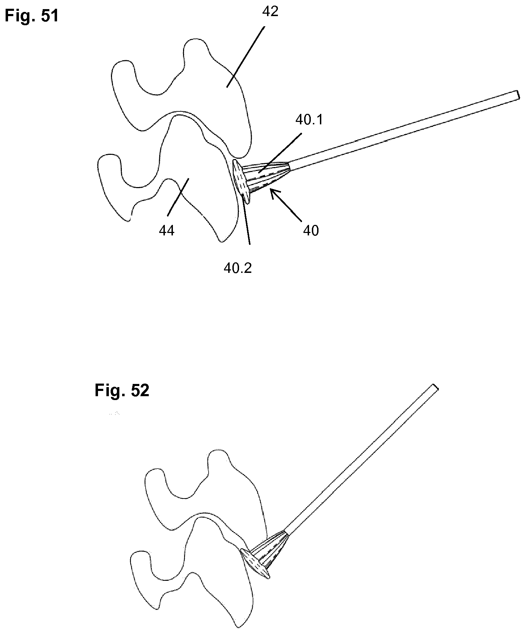

Die Figur 51 bis 55 zeigen verschiedene Ansichten zur Verdeutlichung des Operationsverfahren unter Verwendung eines erfindungsgemäßen Fräsers 40. Der Fräser 40 umfasst eine konisch kontinuierlich sich vom Fräsflächenproximalende bis zum Fräsflächendistalende erweiternde Fräsfläche 40.1 mit verdrallten Schneiden sowie einer diskusförmigen Schutzanordnung 40.2, welche den Fräsflächenmaximalradius am Fräsflächendistalende etwa verdoppelt.

Der Fräser ist in Figur 51 vor dem Beginn des Eingriffs zwischen einer oberen Lamina 42 und eine daran angrenzenden untere Lamina 44 angeordnet. Zunächst schiebt der Chirurg den Fräser 40 seitlich in den interlaminären Spalt zwischen der oberen Lamina 42 und die unteren Lamina 44.

Sodann wird gemäß Figur 52 das untere Ende der oberen Lamina 42 abgefräst, wobei sich die Schutzanordnung 40.2 in dem in dem interlaminären Spalt eindringt, wobei die proximale Anlagefläche und die Distalauflagefläche der Schutzanordnung 40.2 an der oberen Lamina 42 und der unteren Lamina 44 geführt wird.

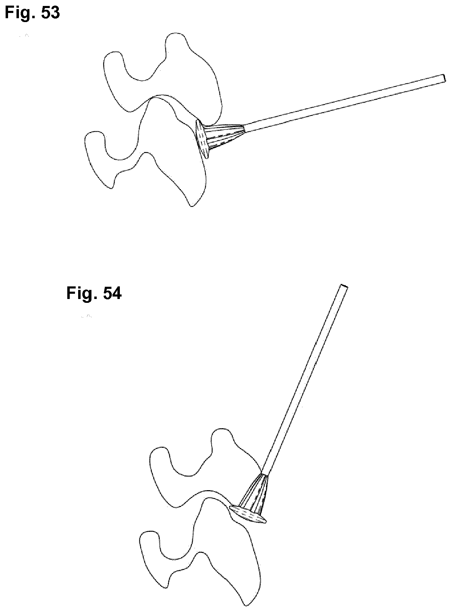

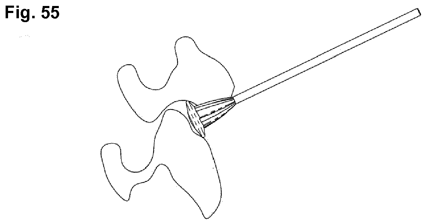

Bei der Darstellung in Figur 53 ist das untere Ende der oberen Lamina 42 abgefräst und der Fraser 40 kann weiter in den interlaminären Spalt eingeschoben werden, um die Schutzanordnung als Hebel einzusetzen und den interlaminären Spalt aufzuweiten und sodann gemäß der Darstellungen in Figur 54 und 55 weiter abfräsen zu können.

Es ist anzumerken, dass die in der Figurenfolge 51 bis 55 dargestellten Figuren übertrieben groß dargestellt sind und nicht die tatsächlichen Abmessungen wiedergeben.

Hinsichtlich der Materialien ist zu bemerken, dass der Fräser bzw. der Fräskopf vorzugsweise aus Edelstahl besteht, was auch bei den Schutzanordnungen der Fall sein können. Diese können aber auch aus Kunststoff, insbesondere Polyetheretherketon (PEEK) oder PTFE/Teflon® bestehen. Bei der Ausbildung der Schutzanordnung als Schutzring, der über eine Mutter an dem Gewindezapfen befestigt wird (Ausführungsform gemäß Figur 42 oder 46) hat sich die Ausbildung des Schutzrings aus Keramik als wirtschaftlich besonders interessant erwiesen, die besonders einfach zu reinigen und häufiger zu verwenden ist.

Ausführungsformen sehen vor, dass sich die Schutzanordnung aufgrund der geometrischen Ausgestaltung, dem Auswahl eines elastischen Materials oder einer Kombination von beiden verformen kann, um sich besser für die anatomischen Verhältnisse (z.B. den engen Spalt) anzupassen.

Dies kann durch Wahl einen elastischen Materials (vorzugsweise PTFE/Teflon®, einer biokompatiblen Silikonart oder eines anderen elastischen biokompatiblen Werkstoffs) und/oder die geometrische Ausgestaltung der Schutzanordnung selbst, insbesondere im Bereich des o.g. Zwischenabschnitts realisiert werden. Diese elastische Verformung kann sowohl die äußere Form der Schutzanordnung als auch die Lage der Schutzanordnung bezüglich der Drehachse und dem Fräsflächenmaximalradius R1 in den allen drei Achsen betreffen. Die Elastizität kann sich dabei sowohl an der äußeren Mantelfläche der Schutzanordnung entfalten oder aber auch durch eine teilweise elastische Verbindung zwischen der Schutzanordnung und dem Fräser bzw. dem Sitz zur Verbindung der Schutzanordnung an dem Fräser umfassen, z.B. durch elastische Ausbildung des Kopplungsmittel , insbesondere der Kopplungskugel.

Obgleich der vorgeschlagene Fräser für die Chirurgie besonders geeignet ist, kann die ausschließlich proximal wirkende Fräsfläche und die distale Schutzanordnung auch in anderen Bereichen der Frästechnik eingesetzt werden.

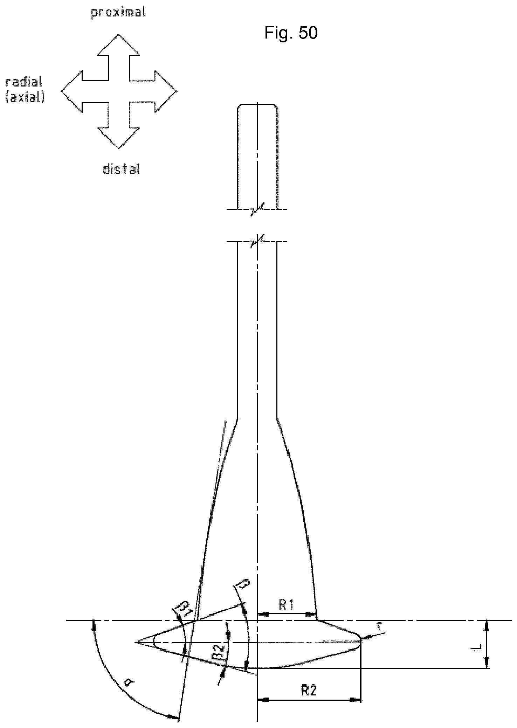

In der Figur 50 sind nochmals deutlich die einzelnen Geometrien / Radien eines bespielhaften, erfindungsgemäßen Fräsers veranschaulicht. Demnach sind:

R1    Fräsflächenmaximalradius

R2    Schutzanordnungsmaximalradius

r    Radius der Abrundung an der radial äußeren Kante am Schutzanordnungsmaximalradius

L    Länge der Schutzanordnung

β    Keilwinkel der Schutzanordnung

β1    Keilwinkel der Proximalfläche der Schutzanordnung

β2    Keilwinkel der Distalfläche der Schutzanordnun

σ    Profilwinkel einer konischen Fräsfläche.

Der Gegenstand der vorliegenden Erfindung ergibt sich aus den Patentansprüchen.

**Bezugszeichenliste**

| | |
|---|---|
| 2 | Schaft |
| 4 | Fräskopf |
| 4.1 | Fräsfläche |
| 4.2 | Schutzring |
| 4.3 | Schutzkappe |
| 6 | Fräskopf |
| 6.1 | Fräsfläche |
| 6.2 | Schutzring |
| 6.3 | Schutzkappe |
| 8 | Gewebe |
| 10 | Knochen |
| 12 | dreidimensionale Schutzzone |
| 14 | Fräskopf |
| 14.1 | Fräsfläche |
| 14.2 | obere Kreisplatte |
| 14.3 | untere Kreisplatte |
| 16 | Schutzkappe |
| 16.1 | Einstecköffnung |
| 16.2 | Rand |
| 18 | Fräskopf |
| 18.1 | Fräsfläche |
| 18.3 | Distalauflagefläche |
| 20 | Schutzring |
| 20.1 | Einstecköffnung |
| 22 | Schutzkappe |
| 22.3 | Kugelkappe |
| 22.4 | Kopplungskugel |
| 22.5 | Sitz |
| 22.6 | Schutzring |
| 24 | Fräskopf |
| 24.1 | Fräsfläche |
| 24.4 | Kreisplatte |
| 24.5 | Einstecköffnung |
| 26 | Fräskopf |
| 26.1 | Fräsfläche |
| 26.3 | Schutzkappe |
| 26.4 | Schutzgürtel |
| 28 | Fräskopf |
| 28.1 | Fräsfläche |
| 28.3 | Schutzkappe |
| 28.4 | Schutzgürtel |
| 30 | Fräskopf |
| 30.1 | Fräsfläche |
| 30.2 | Fräsflächenmaximalradius |
| 30.3 | Einstecköffnung |
| 32 | Schutzkappe |
| 32.4 | Kopplungskugel |
| 34 | Fräskopf |

| | |
|---|---|
| 34.1 | Fräsfläche |
| 34.2 | Schutzring |
| 34.3 | Distalauflagefläche |
| 36 | Fräskopf |
| 36.1 | Fräsfläche |
| 36.2 | Schutzring |
| 36.3 | Distalauflagefläche |
| 38 | Fräskopf |
| 38.1 | Fräsfläche |
| 38.2 | Schutzring |
| 38.3 | Distalauflagefläche |
| 40 | Fräser |
| 40.1 | Fräsfläche |
| 40.2 | Schutzkappe |
| 42 | obere Lamina |
| 44 | untere Lamina |

**Patentansprüche**

1. Chirurgischer Fräser, ausgebildet zum Abtragen von Knochen und/oder Knorpelgewebe, umfassend einen um eine Rotationsachse drehbaren und eine Längsachse definierenden Schaft (2), der einen Nenndurchmesser aufweist und ein drehfest mit einer Antriebseinheit verbindbares Proximalende sowie ein diesem Proximalende gegenübergelegenes Distalende aufweist, wobei an dem Distalende ein Fräskopf (4) mit einer den Schaft (2) umfänglich umschließenden und sich entlang der Längsachse des Schafts (2) erstreckenden Fräsfläche (4.1) ausgebildet ist, wobei die Fräsfläche (4.1) begrenzt ist durch ein proximal gelegenes Fräsflächenproximalende sowie distal gelegenes Fräsflächendistalende und einen Fräsflächenmaximalradius (R1) aufweist, wobei die Fräsfläche auch proximal wirkend ausgebildet ist, wobei die Fräsfläche in distaler Richtung radial über den Nenndurchmesser des Schafts (2) erweitert ausgebildet ist, wobei an dem Distalende eine Schutzanordnung ausgebildet ist, wobei die Schutzanordnung distal von dem Fräsflächendistalende ausgebildet ist und wobei die Schutzanordnung eine Distalauflagefläche aufweist, **DADURCH GEKENNZEICHNET, DASS** der Fräskopf (4) atraumatisch ausgebildet ist, dass die Schutzanordnung einen Schutzanordnungsmaximalradius (R2) zur Bildung eines Schutzrings (4.2) aufweist, der den durch den Fräsflächenmaximalradius (R1) definierten Fräsflächenmaximalumfang kreisringförmig umschließt, so dass die Schutzanordnung bei der Rotation des Fräsers eine sich von dem Schutzanordnungsmaximalradius (R2) bis zum Fräsflächendistalende erstreckende, umfänglich umgebende Schutzzone (12) um die Fräsfläche (4.1) definiert.

2. Chirurgischer Fräser nach Anspruch 1, **DADURCH GEKENNZEICHNET, DASS** sich der Schutzring (4.2) radial nach außen verjüngt.

3. Chirurgischer Fräser nach Anspruch 2, **DADURCH GEKENNZEICHNET, DASS** der Schutzring (4.2) keilförmig ausgebildet ist.

4. Chirurgischer Fräser nach Anspruch 3, **DADURCH GEKENNZEICHNET, DASS** der Schutzring (4.2) eine proximale Keilfläche umfasst, die mit einer sich durch den Fräsflächenmaximalradius (R1) erstreckenden Geraden einen proximalen Keilwinkel ($\beta_1$) einschließt.

5. Chirurgischer Fräser nach Anspruch 3, **DADURCH GEKENNZEICHNET, DASS** der Schutzring (4.2) eine distale Keilfläche umfasst, die mit einer sich durch den Fräsflächenmaximalradius (R1) erstreckenden Geraden einen distalen Keilwinkel ($\beta_2$) einschließt.

6. Chirurgischer Fräser nach Anspruch 4 und 5, **DADURCH GEKENNZEICHNET, DASS** der Schutzring (4.2) einen proximalen Keilwinkel ($\beta_1$) und einen distalen Keilwinkel ($\beta_2$) umfasst.

7. Chirurgischer Fräser nach einem der vorhergehenden Ansprüche, **DADURCH GEKENNZEICHNET, DASS** die Fräsfläche auch proximal wirkend ausgebildet ist.

8. Chirurgischer Fräser nach einem der vorhergehenden Ansprüche, **DADURCH GEKENNZEICHNET, DASS** der Schutzanordnungsmaximalradius (R2) mindestens dem Fräsflächenmaximalradius (R1) zuzüglich einer oberen, vorgegebenen Grenzabweichung zuzüglich das Zehnfache eines vorgegebenen Rundlaufs beträgt.

**9.** Chirurgischer Fräser nach einem der vorhergehenden Ansprüche, **DADURCH GEKENNZEICHNET, DASS** die Schutzanordnung eine Schutzkappe (4.3) umfasst, die die Distalauflagefläche bildet und sich bis zum Schutzanordnungsmaximalradius (R2) erstreckt.

**10.** Chirurgischer Fräser nach einem der vorhergehenden Ansprüche, **DADURCH GEKENNZEICHNET, DASS** die Schutzanordnung einen Schutzgürtel (26.4; 28.4) umfasst, der sich in proximaler Richtung über den Schutzanordnungsmaximalradius (R2) erstreckt.

**11.** Chirurgischer Fräser nach einem der vorhergehenden Ansprüche, **DADURCH GEKENNZEICHNET, DASS** die Schutzanordnung über Verbindungsmittel mit dem Fräskopf (4) verbindbar ist.

**12.** Chirurgischer Fräser nach Anspruch 11, **DADURCH GEKENNZEICHNET, DASS** die Verbindungsmittel eine Schraubverbindung umfassen.

**13.** Chirurgischer Fräser nach Anspruch 12, **DADURCH GEKENNZEICHNET, DASS** die Schraubverbindung einen distal von der Fräsfläche ausgebildeten Gewindestift umfassen, der mit der Schutzanordnung verbindbar ist.

**14.** Chirurgischer Fräser nach einem der vorhergehenden Ansprüche, **DADURCH GEKENNZEICHNET, DASS** die Schutzanordnung widerstandsreduzierend ausgebildet ist.

**15.** Chirurgischer Fräser nach einem der vorhergehenden Ansprüche, **DADURCH GEKENNZEICHNET, DASS** die Schutzanordnung zumindest teilweise elastisch ausgebildet ist.

**Claims**

**1.** A surgical milling cutter designed for the removal of bone and/or cartilage tissue comprising a shaft (2) which defines a longitudinal axis and which is rotatable about an axis of rotation and which has a nominal diameter and a proximal end which can be non-rotatably connected to a drive unit and a distal end opposite the proximal end, wherein provided at the distal end is a milling head (4) having a milling surface (4.1) which circumferentially surrounds the shaft (2) and extends along the longitudinal axis of the shaft (2), wherein the milling surface (4.1) is delimited by a proximally disposed milling surface proximal end and a distally disposed milling surface distal end and a milling surface maximum radius (R1), wherein the milling surface is also proximally operative, the milling surface is enlarged in the distal direction radially beyond the nominal diameter of the shaft, that a protective arrangement is provided at the distal end, the protective arrangement is provided distally from the milling surface distal end, the protective arrangement has a distal contact surface, **characterised in that** the milling head (4) is of an atraumatic configuration, that the protective arrangement is provided with a protective arrangement maximum radius (R2) for forming a protective ring (4.2) which surrounds the milling surface maximum radius (R1) in an annular configuration so that the protective arrangement upon rotation of the milling cutter defines around the milling surface (4.1) a circumferentially surrounding protective zone (12) extending from the protective arrangement maximum radius (R2) to the milling surface distal end.

**2.** A surgical milling cutter according to claim 1 **characterised in that** the protective ring (4.2) narrows radially outwardly.

**3.** A surgical milling cutter according to claim 2 **characterised in that** the protective ring (4.2) is wedge-shaped.

**4.** A surgical milling cutter according to claim 3 **characterised in that** the protective ring (4.2) includes a proximal wedge surface which includes a proximal wedge angle ($\beta_1$) with a straight line extending through the milling surface maximum radius (R1).

**5.** A surgical milling cutter according to claim 3 **characterised in that** the protective ring (4.2) includes a distal wedge surface which includes a distal wedge angle ($\beta_2$) with a straight line extending through the milling surface maximum radius (R1).

**6.** A surgical milling cutter according to claim 4 and claim 5 **characterised in that** the protective ring (4.2) includes a

proximal wedge angle ($\beta_1$) and a distal wedge angle ($\beta_2$).

7. A surgical milling cutter according to one of the preceding claims **characterised in that** the milling surface is also proximally operative.

8. A surgical milling cutter according to one of the preceding claims **characterised in that** the protective arrangement maximum radius (R2) corresponds at least to the milling surface maximum radius (R1) plus an upper predetermined limit deviation plus ten times the predetermined concentricity.

9. A milling cutter according to one of the preceding claims **characterised in that** the protective arrangement includes a protective cap (4.3) which forms a distal contact surface and extends to the protective arrangement maximum radius (R2).

10. A milling cutter according to one of the preceding claims **characterised in that** the protective arrangement includes a protective girdle (26.4; 28.4) which extends in the proximal direction over the protective arrangement maximum radius (R2).

11. A milling cutter according to one of the preceding claims **characterised in that** the protective arrangement can be connected to the milling head (4) by way of connecting means.

12. A milling cutter according to claim 11 **characterised in that** the connecting means include a screw connection.

13. A milling cutter according to claim 12 **characterised in that** the screw connection includes a threaded pin provided distally from the milling surface and which can be connected to the protective arrangement.

14. A milling cutter according to one of the preceding claims **characterised in that** the protective is of a resistance-reducing nature.

15. A milling cutter according to one of the preceding claims **characterised in that** the protective arrangement is at least partially elastic.


**Revendications**

1. Fraise chirurgicale, destinée à l'enlèvement des os et/ou de tissu cartilagineux, comprenant une tige (2) pouvant tourner autour d'un axe de rotation et définissant un axe longitudinal, présentant un diamètre nominal et une extrémité proximale pouvant être reliée de manière fixe en rotation à une unité d'entraînement et présentant une extrémité distale opposée à cette extrémité proximale, une tête de fraisage (4) présentant une surface de fraisage (4.1) entourant la tige (2) de manière circonférentielle et s'étendant le long de l'axe longitudinal de la tige (2) étant formée à l'extrémité distale, la surface de fraisage (4.1) étant limitée par une extrémité proximale de surface de fraisage située proximalement et par une extrémité distale de surface de fraisage située distalement et présentant un rayon maximal de surface de fraisage (R1), la surface de fraisage étant conçue pour agir proximalement, la surface de fraisage étant conçue de manière élargie radialement sur le diamètre nominal de la tige (2) dans la direction distale, un dispositif de protection étant formé à l'extrémité distale, le dispositif de protection étant formé distalement de l'extrémité distale de surface de fraisage, et le dispositif de protection ayant une surface d'appui distale, **caractérisée en ce que** la tête de fraisage (4) est formée de façon atraumtique, que le dispositif de protection présente un rayon maximal (R2) du dispositif de protection (R2) pour former une bague de protection (4.2) qui entoure la circonférence maximale de la surface de fraisage définie par le rayon maximal (R1) de la surface de fraisage sous la forme d'une bague circulaire, de sorte que, pendant la rotation de la fraise, le dispositif de protection définit une zone de protection (12) circonférentiellement autour de la surface de fraisage (4.1), laquelle zone de protection s'étendant du rayon maximal (R2) du dispositif de protection jusqu'à l'extrémité distale de la surface de fraisage.

2. Fraise chirurgicale selon la revendication 2, **caractérisée en ce que** la bague de protection (4.2) diminue radialement vers l'extérieur.

3. Fraise chirurgicale selon la revendication 2, **caractérisée en ce que** la bague de protection (4.2) est en forme de coin.

4. Fraise chirurgicale selon la revendication 3, **caractérisée en ce que** la bague (4.2) comprend une surface en coin proximale incluant un angle de coin proximale ($\beta_1$) avec une ligne droite s'étendant à travers le rayon maximal (R1) de la surface de fraisage.

**5.** Fraise chirurgicale selon la revendication 3, **caractérisée en ce que** la bague (4.2) comprend une surface en coin distale incluant un angle de coin distale ($\beta_2$) avec une ligne droite s'étendant à travers le rayon maximal (R1) de la surface de fraisage.

**6.** Fraise chirurgicale selon la revendication 3, **caractérisée en ce que** la bague (4.2) comprend un angle de coin proximale ($\beta_1$) et un angle de coin distale ($\beta_2$).

**7.** Fraise chirurgicale selon l'une des revendications précédentes, **caractérisée en ce que** la surface de fraisage est aussi conçue pour agir proximalement.

**8.** Fraise chirurgicale selon l'une des revendications précédentes, **caractérisée en ce que** le rayon maximal (R2) du dispositif de protection est au moins le rayon maximal (R1) de la surface de fraisage plus un écart limite supérieur prédéterminé plus dix fois une concentricité prédéterminée.

**9.** Fraise chirurgicale selon l'une des revendications précédentes, **caractérisée en ce que** le dispositif de protection comprend un capuchon de protection (4.3) formant la surface d'appui distale et s'étendant jusqu'au rayon maximal (R2) due dispositif de protection.

**10.** Fraise chirurgicale selon l'une des revendications précédentes, **caractérisée en ce que** le dispositif de protection comprend une ceinture de protection (26.4; 28.4) qui s'étend dans la direction proximale sur le rayon maximal (R2) du dispositif de protection.

**11.** Fraise chirurgicale selon l'une des revendications précédentes, **caractérisée en ce que** le dispositif de protection peut être relié à la tête de fraisage (4) par des moyens de raccordement.

**12.** Fraise chirurgical selon la revendication 11, **caractérisée en ce que** les moyens de raccordement comportent un raccordement vissé.

**13.** Fraise chirurgical selon la revendication 12, **caractérisée en ce que** le raccordement vissé comprend un goujon fileté formé distalement de la surface de fraisage et pouvant être relié au dispositif de protection.

**14.** Fraise chirurgicale selon l'une des revendications précédentes, **caractérisée en ce que** le dispositif de protection est conçu pour réduire la résistance.

**15.** Fraise chirurgicale selon l'une des revendications précédentes, **caractérisée en ce que** le dispositif de protection est au moins partiellement élastique.

Fig. 1

Fig. 2

4.1

4.2

A

A

Fig. 3

A–A

Fig. 4

2

4

4.1

4.2

4.3

Fig. 5

2

6

6.1

Fig. 6

B

B

Fig. 7

2

B–B

6

2

Fig. 8

6.1

6

6.2

6.3

Fig. 9

Fig. 10

14.1

14.4

14.5

16.2

16.1

16

Fig. 11

14

14.1

14.2

14.3

Fig. 12

2

14

16

Fig. 13

2

14

16

Fig. 14

2

16

Fig. 18

Fig. 16

18.1

18

18.3

20

Fig. 15

2

18.1

20.1

20

Fig. 17

2

20

2

20

Fig. 20

Fig. 23

Fig. 19

2

24.1

24 →

24.4

24.5

22.3

22

2

22.5

22.6

22.4

Fig. 22

2

Fig. 21

24.1

22

2

24.1

2

22.4

22.6

22.3

22

Fig. 24     Fig. 25

2

26

26.1

26.4

26.3

28

28.1

28.4

28.3

2

Fia. 26

Fia. 27

Fia. 31

30

2

30.1

30.2

32

30.3

32.4

Fig. 28

Fig. 29

2

Fia. 30

32.4

32

Fig. 32

Fig. 34a

34.1

34

34.2

34.3

Fig. 33

36.1

36

36.2

36.3

10°

Fig. 34

38

38.1

38.2

38.3

20°

Fig. 35

Fig. 36

Fig. 38

Fig. 37

Fig. 39

Fig. 40

Fig. 41

Fig. 42

Fig. 43

Fig. 44

Fig. 45

Fig. 46

Fig. 47

Fig. 48

Fig. 49

Fig. 50

**Fig. 51**

42

40.1

44

40.2

40

**Fig. 52**

**Fig. 53**

**Fig. 54**

**Fig. 55**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015009810 A
- DE 10022047 C1
- US 2004133209 A1
- DE 102004040581 A1

**In der Beschreibung aufgeführte Nicht-Patentliteratur**

- **GUERIN P.** *Injury, Int. J. Care Injured,* 2012, vol. 43, 397-401
- **GOODKIN.** *Surg Neurol,* 1995, vol. 43, 4-14